Europäisches Patentamt

European Patent Office

Office européen des brevets

(19)

(11) **EP 1 232 377 B1**

(12) **EUROPÄISCHE PATENTSCHRIFT**

(45) Veröffentlichungstag und Bekanntmachung des Hinweises auf die Patenterteilung:
**31.03.2004 Patentblatt 2004/14**

(21) Anmeldenummer: **00975736.0**

(22) Anmeldetag: **24.11.2000**

(51) Int Cl.⁷: $G01B\ 9/02$, $G01B\ 11/06$, $G01N\ 21/45$

(86) Internationale Anmeldenummer:
**PCT/CH2000/000634**

(87) Internationale Veröffentlichungsnummer:
**WO 2001/038820 (31.05.2001 Gazette 2001/22)**

(54) **VERFAHREN UND VORRICHTUNG ZUR MESSUNG OPTISCHER EIGENSCHAFTEN WENIGSTENS ZWEIER VONEINANDER DISTANZIERTER BEREICHE IN EINEM TRANSPARENTEN UND/ODER DIFFUSIVEN GEGENSTAND**

METHOD AND DEVICE FOR MEASURING THE OPTICAL PROPERTIES OF AT LEAST TWO REGIONS LOCATED AT A DISTANCE FROM ONE ANOTHER IN A TRANSPARENT AND/OR DIFFUSE OBJECT

PROCEDE ET DISPOSITIF POUR MESURER LES PROPRIETES OPTIQUES D'AU MOINS DEUX SECTEURS DISTANTS L'UN DE L'AUTRE DANS UN OBJET TRANSPARENT ET/OU DIFFUSANT

(84) Benannte Vertragsstaaten:
**AT BE CH CY DE DK ES FI FR GB GR IE IT LI LU MC NL PT SE TR**

(30) Priorität: **24.11.1999 CH 214699**

(43) Veröffentlichungstag der Anmeldung:
**21.08.2002 Patentblatt 2002/34**

(73) Patentinhaber: **HAAG-STREIT AG CH-3098 Köniz (CH)**

(72) Erfinder:
• **WAELTI, Rudolf CH-3097 Liebefeld (CH)**

• **SCHMID, Gregor, F. 8360 Old York Road Elkins Park, Pa 19027 (US)**

(74) Vertreter: **Roshardt, Werner Alfred, Dipl.-Phys. Keller & Partner Patentanwälte AG Schmiedenplatz 5 Postfach 3000 Bern 7 (CH)**

(56) Entgegenhaltungen:
**EP-A- 0 671 601        EP-A- 0 932 021 WO-A-96/35100**

## Beschreibung

### Technisches Gebiet

Die Erfindung betrifft ein Verfahren, eine Vorrichtung zur Messung optischer Eigenschaften wenigstens zweier voneinander distanzierter Bereiche bei einem transparenten und/oder diffusiven Gegenstand sowie deren Verwendung zur Dicken-, Abstands- und/oder Profilmessung. Die Dicken-, Abstands- und/oder Profilmessung wird mittels Kurzkohärenzreflektometrie vorgenommen.

Die Transparenz von Gegenständen hängt von deren wellenlängenabhängigen Schwächungskoeffizienten $\alpha$ [cm$^{-1}$] und deren Dicke bzw. der vorgegebenen Messstrecke d ab. Als transparent werden Gegenstände bezeichnet, deren Transmissionsfaktor T = exp (-$\alpha$·d) noch innerhalb des Messbereichs der unten beschriebenen Interferometer liegt, wobei die Transmission T$^2$ bei den unten beschriebenen Interferometern infolge des "Hin- und Rückweges" der Strahlung ist. In diffusiven Gegenständen wird die Strahlung stark gestreut, nicht notwendigerweise absorbiert. Beispielsweise sind als diffusive Gegenstände Milchglasscheiben, Delrin, organische Gewebe (Haut, menschliche und tierische Organe, Pflanzenteile etc.) anzusehen.

Eine Kurzkohärenzreflektometrie wurde in der Regel für präzise, rasche und nicht-invasive Abbildungen vorgenommen. Typischerweise wurde in einer optischen Anordnung mit einem Michelson-Interferometer der Strahl einer Strahlungsquelle in einen Referenz- und einen Messstrahl mit einem Strahlteiler aufgespalten. In der Regel wurde eine Strahlungsquelle mit einer kurzen Kohärenzlänge ausgewählt. Eine Aufspaltung in Referenz- und Messstrahl sowie deren Wiedervereinigung erfolgte mit einem Strahlteiler und bei Verwendung von Faseroptikwegen mit einem Faserkoppler. Die optische Weglängenänderung im Referenzarm konnte durch Verschieben eines Referenzspiegels auf einer Translationsbühne erreicht werden. Vorteilhafterweise verwendete man jedoch einen rotierenden transparenten Würfel, wie er in der WO96/35100 beschrieben war. Nur wenn der Weglängenunterschied kleiner als die Kohärenzlänge der Strahlung der Strahlungsquelle war, entstand nach der Wiedervereinigung des reflektierten Referenz- und Messstrahls ein Interferenzmuster. Das Interferenzmuster wurde auf einen Photodetektor gebracht, welcher die Strahlungsintensität während der Veränderung der Spiegelposition maß. Da die Frequenz der Strahlung des reflektierten Referenzstrahls wegen der Spiegelverschiebung eine Dopplerverschiebung erfuhr, konnte das Interferenzsignal mit elektronischen Mitteln, wie beispielsweise in der WO99/22198 beschrieben, durch Erhöhung des Signal-Rauschverhältnisses ausgewertet werden.

Messfehler traten jedoch auf, wenn Abstände, welche wenigstens zwei Messvorgänge notwendig machten, in optisch transparenten bzw. eine optische Strahlung diffus transmittierenden Gegenständen gemessen werden sollten und die Gegenstände über dem gesamten Messzyklus innerhalb der geforderten Messtoleranz nur schwer oder nicht ausreichend fixiert werden konnten. Diese Probleme traten insbesondere bei "in-vivo"-Messungen auf.

### Darstellung der Erfindung

### Aufgabe der Erfindung

Aufgabe der Erfindung ist es, ein Verfahren vorzustellen sowie eine Vorrichtung zu schaffen, mit dem bzw. mit der insbesondere "in-vivo"-Messungen von Abständen, Dicken, Oberflächenverläufen,..., welche Messungen an unterschiedlichen Orten in einem Gegenstand beinhalten, optimal d.h. mit einer Messfehlerminimierung durchführbar sind.

### Lösung der Aufgabe

Die Aufgabe wird dadurch gelöst, dass zur Messung optischer Eigenschaften mit einer Messzeit im Subsekundenbereich (notwendig für eine "in-vivo"-Messung) wenigstens zweier voneinander distanzierter Bereiche bei einem transparenten und/oder diffusiven Gegenstand, wie zur Abstands-, Längen-, Dicken- und Profilmessung notwendig, der Gegenstand mit einer der Anzahl Bereiche entsprechenden Anzahl Messstrahlen gleichzeitig oder kurz nacheinander bestrahlt wird. Durch den Ausdruck "bei" einem Gegenstand soll ausgedrückt werden, dass die Bereiche sich an Orten im Gegenstand sowie auch auf dem Gegenstand, z. B. seitlich versetzt befinden können. Jeweils zwei Messstrahlen haben bis auf eine Bestimmungstoleranz zueinander eine optische Wegdifferenz, d. h. einen Laufzeitunterschied. Der Laufzeitunterschied entspricht einem optischen Abstand zweier Raumpunkte (Bereiche) in Bezug auf die Ausbreitungsrichtung der Messstrahlung, wobei wenigstens einer der Raumpunkte wenigstens geringfügig (typischerweise mindestens 10$^{-4}$% der Strahlungsintensität) reflektiert. Die Messstrahlen können somit übereinander liegen (Dicken-, Distanz-, Längenmessung), parallel zueinander verlaufen (Oberflächenprofil, ...) oder beliebige Winkel zueinander aufweisen (Dicken-, Distanzmessung, ... bei vorgegebenem Winkel zu einer Referenzfläche). Jeder von einem der Bereiche reflektierte Reflexionsstrahl der Messstrahlen wird mit einem eine zeitliche, bevorzugt periodische, Weglängenvariation aufweisenden dritten Strahl interferierend überlagert detektiert.

[0007] - Nach der Vornahme der Wegdifferenz bzw. -differenzen erfolgt vorzugsweise zur Dickenmessung eine Vereinigung der Messstrahlen zu einer einzigen Strahlkonfiguration mit einer einzigen optischen Achse. Auch kann die Strahlkonfiguration über den Gegenstand, insbesonders periodisch bewegt werden. Es erfolgt hierdurch ein seitliches Abscannen. Dieses Abscannen unter einem Abspeichem der ermittelten Werte kann zur Erstellung von Profilen dienen. Anstelle die beiden Messstrahlen auf einer optischen Achse zu fokussieren, können jedoch auch jeweils wenigstens zwei Messstrahlen in einem Abstand nebeneinander verlaufen und fokussiert werden, um ein Oberflächenprofil zu ermitteln.

Die Messstrahlen haben im Vergleich zu den Bereichsabsländen, insbesondere zu den Bereichsabständen ausgehend von einem Referenzort eine kurze Kohärenzlänge. Die Messstrahlen können ferner jeweils sich voneinander unterscheidende Strahlungsfrequenzen haben. Es müssen dann jedoch mehrere Strahlungsquellen verwendet werden. Man kann auch mit nur einer Strahlungsquelle arbeiten und über Filter eine Aufteilung vornehmen. Hierbei ergibt sich jedoch ein Verlust an Breitbandigkeit; auch müssen einige der Komponenten mit einer aufwendigen Beschichtung versehen werden.

Anstelle von unterschiedlichen Strahlungsfrequenzen bzw. ergänzend hierzu können die Messstrahlen sich voneinander unterscheidende Polarisationszustände haben, was einen einfacheren Aufbau ergibt. Vorzugsweise wird man auch eine Fokussierung der Messstrahlen in den auszumessenden Bereich bzw. die auszumessenden Bereiche vornehmen. Da mit einer "Michelson-Interferometer"-artigen optischen Anordnung gearbeitet wird, kann die augenblickliche Position des reflektierenden Elements im Referenzarm als Referenzort dienen. Es kann nun die tatsächliche Position hierzu verwendet werden oder ein mit dem Referenzort gekoppelter anderer Wert, wie beispielsweise die Verdrehungsposition des in der WO 96/35100 beschriebenen rotierenden Würfels.

Die Messung wird mit einer "Michelson-Interferometer"-artigen optischen Anordnung, in deren Messarm der optisch transparente und/oder diffusive Gegenstand einbringbar ist, durchgeführt. Anstelle eines optisch transparenten und/oder diffusiven Gegenstands kann auch mit einem Gegenstand gearbeitet werden, dessen Oberfläche reflektierend ist. Bei einem reflektierenden Gegenstand kann mit der erfindungsgemäßen Methode insbesondere dessen Oberflächenprofil ermittelt werden. Der Gegenstand kann jedoch optisch transparent und/oder diffusiv sein und eine (wenigstens einige wenige Prozente) reflektierende Oberfläche haben. In diesem Fall können dann sowohl Oberflächen, wie auch Dicken bzw. deren Verläufe ermittelt werden. Der Referenzarm weist eine Weglängenvariationseinheit auf, mit der bevorzugt eine periodische Weglängenänderung im Referenzarm durchführbar ist. Im Messarm ist vor dem Gegenstand eine Umwegeinheit angeordnet, mit der wenigstens ein erster Messstrahl mit einer gegenüber wenigstens einem zweiten Messstrahl größeren Laufzeit beaufschlagbar ist, wobei ein mit der Umwegeinheit erzeugbarer Umweg bis auf eine Bestimmungstoleranz gleich einem Abstand von mindestens zweier im Gegenstand auszumessenden Bereichen wählbar ist. Anstelle der im Gegenstand "hintereinander" liegenden Bereichen (Orte) zur Dickenmessung, können selbstverständlich auch "nebeneinander" liegende Bereich (Ort) zur Bestimmung von Oberflächenkrümmungen, bzw. Oberflächenverläufen ausgemessen werden.

Der Umweg wird näherungsweise derart eingestellt, dass er einem zu erwartenden Messergebnis einer zu bestimmenden Dicke, Abstand, ... bis auf eine Bestimmungstoleranz entspricht. Mit der Wegvariationseinheit im Referenzarm muss dann nur noch der nicht bekannte (zu bestimmende) Anteil der Dicke, des Abstands etc. ermittelt werden. Soll z.B. die tatsächliche Länge eines menschlichen Auges bestimmt werden, so weiß man ja bereits im Vorhinein, dass Augen eine optische Länge von 34 mm mit einer Längentoleranz von +/- 4 mm aufweisen. Es kann nun hier ein Umweg auf 34 mm eingestellt und mit der Wegvariationseinheit eine Variation von lediglich 8 mm vorgenommen werden.

Mit der unten beschriebenen Vorrichtung und deren Ausführungsvarianten kann am Auge neben der Augenlänge, die Corneadicke, die Vorderkammertiefe, die Linsendicke und die Glaskörpertiefe sowie entsprechende Oberflächenprofile gemessen werden. Hierzu wird der für die Augenoberfläche als Gegenstandsoberfläche bestimmte Messstrahl "irgendwo" zwischen der Corneavorderseite und der Linsenrückseite fokussiert. Durch diesen "Kompromiss" kann dann die Reflexion an der Corneavorderseite, der Cornearückseite, der Linsenvorderseite und der Linsenrückseite detektiert werden. Die Distanz zwischen der Cornearückseite und der Linsenvorderseite ist dann die Vorderkammertiefe. Bedingung für diese Messung ist jedoch, dass der "optische" Hub (ca. 8 mm) der Wegvariationseinheit derart groß ist, dass von der Corneavorderseite bis zur Linsenrückseite gescannt werden kann.

Eine einzige Messung verarbeitet somit die Reflexionen an mehreren Bereichen zusammen nahezu gleichzeitig. Um die einzelnen Reflexionen messtechnisch dennoch unterscheiden zu können, haben die Messstrahlen unterschiedliche optische Eigenschaften, wie unterschiedliche Polarisationsrichtung, unterschiedliche Wellenlänge, ... . Man kann jedoch auch mit nicht unterscheidbaren Strahlen arbeiten und durch eine Veränderung des Umwegs die beiden Interferenzsignale zur Deckung bringen. In diesem Fall ist dann der eingestellte Umweg gleich dem gesuchten Abstand, Dicke etc. Die Verwendung von nicht unterscheidbaren Strahlen führt zu einem Sensitivitätsverlust.

Je nach Anzahl verwendeter Messstrahlen können ein oder mehrere Abstände mit einer Messung bestimmt werden. Die Weglängenänderungen im Referenzarm können, wie in der WO 96/35100 beschrieben, mit einem rotierenden transparenten Würfel vor einem feststehenden Reflektor vorgenommen werden. Ein derartiger Würfel kann problemlos mit über 10 Hz rotieren. D.h. bei den meisten Messungen kann der auszumessende Gegenstand, ohne dass besondere Vorkehrungen für seine Fixierung vorgesehen werden, als in Ruhe befindlich betrachtet werden.

Der Umweg wird mit einer Umwegeinheit erzeugt, in der die geometrisch-optische Länge des Umwegs durch eine Abstandsverstellung eines Umlenkspiegels gegenüber einem der Strahlteiler veränderbar ist. Der Strahlteiler und jeder diesem zugeordnete Umlenkspiegel sind insbesondere derart zueinander ausgerichtet, dass jeder umgelenkte Messstrahl mit dem nicht umgelenkten eine einzige optische Achse innerhalb des Gegenstands hat sowie wahlweise je eine Fokussiereinheit für jeden Messstrahl, um diesen auf je einen Bereich fokussieren zu können. Eine derartige Anordnung kann zur Dicken-, Längen- und/oder Distanzmessung bzw. zur Messung eines Dicken-, Längen- und/oder Distanzprofils verwendet werden.

In der Vorrichtung wird man vorzugsweise eine Speichereinheit verwenden, in der Weglängen der Weglängenvariationseinheit abspeicherbar sind, bei denen eine Interferenz der ersten und dritten sowie zweiten und dritten Messstrahlung abspeicherbar sind. Man wird dann einen Dicken-, Längen- und/oder Distanzwert bei vorzugsweise annähernd auf einer Achse fokussierten ersten und zweiten Messstrahlen bzw. wahlweise ein Oberflächenprofil bei seitlich benachbart liegenden ersten und zweiten Strahlen aus den abgespeicherten Daten ermitteln.

Weitere Ausführungsvariationen zur Erfindung und deren Vorteile ergeben sich aus dem nachfolgenden Text. Allgemein sei bemerkt, das die nachfolgend mit Strahlteiler bezeichneten optischen Einheiten eine Strahlteilung aber auch ein Zusammenfügen von zwei Strahlen vornehmen können.

## Kurze Beschreibung der Zeichnungen

Nachfolgend werden Beispiele der erfindungsgemässen Vorrichtung, mit der das erfindungsgemäße Verfahren durchführbar ist, anhand von Zeichnungen näher erläutert. Es zeigen:

Fig. 1    ein optisches Blockschaltbild einer erfindungsgemässen Vorrichtung,

Fig. 2    eine Variante zur optischen Anordnung in **Figur 1**,

Fig. 3    eine weitere Variante zu den in den **Figuren 1** und **2** dargestellten Vorrichtungen, welche hier jedoch mit zwei unterschiedlichen Zentrumswellenlängen arbeitet,

Fig. 4    ein optisches Blockschaltbild analog **Figur 1**, jedoch zur Ermittlung der Phasenverzögerung in einem doppelbrechenden Material,

Fig. 5    eine Variante zu den in den **Figuren 1** bis **4** dargestellten Vorrichtungen,

Fig. 6    eine Hilfsdarstellung zur Erklärung einer Ermittlung eines Oberflächenprofils,

Fig. 7    ein optisches Blockschaltbild einer Ausführungsvariante zur Ermittlung eines Oberflächenprofils zusammen mit diversen Dicken,

Fig. 8    eine vergrößerte Darstellung des in **Figur 7** gezeigten Strahlenganges unmittelbar vor sowie im Auge **147**,

Fig. 9    eine schematische Darstellung des in **Figur 7** dargestellten Strahlenganges lediglich für die Randstrahlen der auf die Detektoren **172a** und **172c** fallenden Strahlen,

Fig. 10    ein optisches Blockschaltbild einer Variante der erfindungsgemäßen Vorrichtungen, bei der die Strahlung größten Teils in Strahlungsleitern verläuft, wobei die Augenoberfläche zur Darstellung der Auftrefforte der Strahlen um 90° verdreht gezeichnet ist,

Fig. 11    eine schematische Darstellung eines Stereomikroskops eines Spaltlampengeräts mit einem Messstrahlengang im Mittelkanal des Mikroskops und

Fig. 12    eine Spaltlampengerät mit einem auf das Mikroskop aufsteckbaren Adapter.

## Wege zur Ausführung der Erfindung

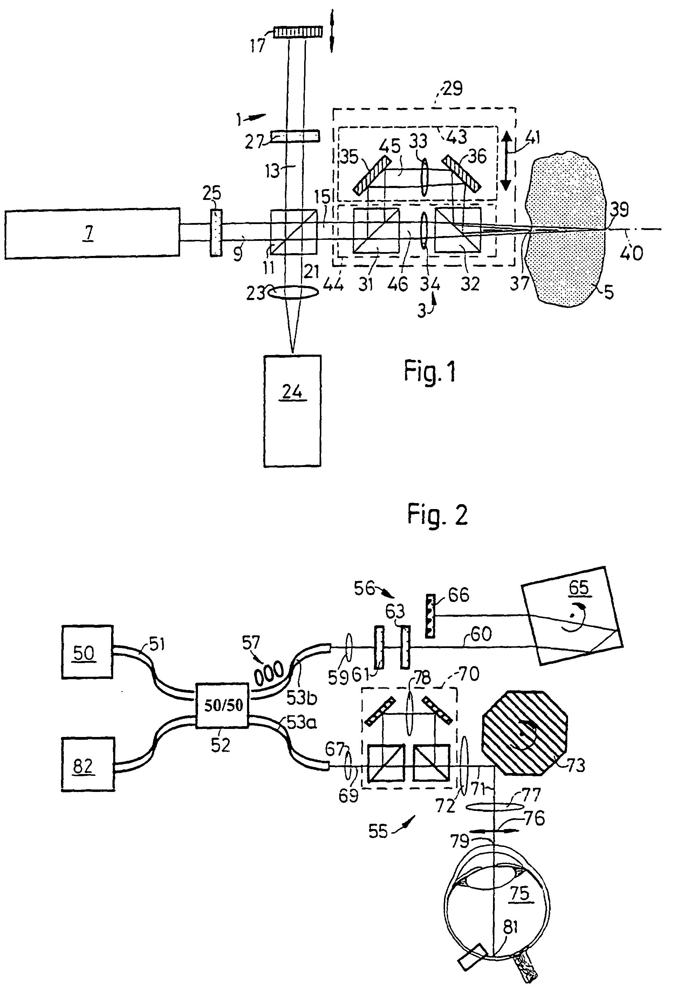

In **Figur 1** ist ein Ausführungsbeispiel der erfindungsgemässen Vorrichtung mit einer "Michelson-Interferometer"-artigen optischen Anordnung dargestellt. Die optische Anordnung hat einen Referenzarm **1** und einen Messarm **3**, in dem der auszumessenden Gegenstand **5** angeordnet ist. Eine Strahlungsquelle **7** sendet einen parallelen Quellenstrahl **9** mit einer linear polarisierten kurzkohärenten Strahlung aus. Die Kohärenzlänge der Strahlung ist kürzer gewählt als die unten beschriebenen, auszumessenden Abstände im Gegenstand **5**. Der von der Strahlungsquelle **7** ausgehende Quellenstrahl **9** wird mit einem die Strahlungspolarisation nicht verändernden physikalischen Strahlteiler **11** in einen Referenz- und einen Gegenstandsstrahl **15** für den Messarm **3** aufgeteilt. Bei einem physikalischen Strahlteiler bleibt gegenüber einem geometrischen der ursprüngliche Strahlquerschnitt erhalten. Es wird die Lichtleistung aufgeteilt, wodurch sich auch dessen Strahldichte ändert. Der Referenzstrahl **13** wird an einer nur schematisch dargestellten, als translatorisch verschiebbaren Spiegel **17** ausgebildeten Weglängenvariationseinheit in sich selbst rückreflektiert. Im Strahlteiler **11** wird er dann mit vom Gegenstand **5** reflektierter Strahlung interferierend überlagert. Diese interferierende Strahlung **21** wird durch Fokussierung mit einer Fokussiereinheit **23** von einem Photodetektor **24** detektiert. Eine andere beispielsweise verwendbare Wegvariationseinheit **17** ist als im Hertzbereich rotierender transparenter Würfel in der WO96/35100 beschrieben und schematisch in **Figur 2** in dem dort gezeigten optischen Aufbau integriert. Anstelle eines rotierenden Würfels können selbstverständlich auch andere Weglängenvariationseinheiten verwendet werden. Es kann beispielsweise auch der hier gezeigte translatorisch "hin- und herwackelnde" Spiegel eingesetzt werden. Dieser "wackelnde" Spiegel könnte eine mit einer Spiegelbeschichtung versehene Lautsprechermembran sein. Im Quellenstrahl **9** ist zwischen Strahlungsquelle **7** und Strahlteiler **11** ein Element **25** zur Drehung der Polarisationsrichtung des Quellenstrahls **9** angeordnet. Ein weiteres Element **27** zur Drehung der Polarisationsrichtung ist zwischen Strahlteiler **11** und Weglängenvariationseinheit **17** angeordnet. Die Elemente **25** und **27** sind hier als sogenannte λ/2- bzw. λ/4 Platten (Viertelwellenlängen-Platte) ausgebildet.

Im Messarm **3** ist vor dem Gegenstand **5** eine Umwegeinheit **29** eingesetzt. Die Umwegeinheit **29** hat zwei polarisationssensitive Strahlteiler **31** und **32,** zwei Fokussiereinheiten, welche symbolisch durch die Linsen **33** und **34** dargestellt sind, sowie zwei Umlenkspiegel **35** und **36.** Da in dem hier ausgeführten Beispiel die Tiefe des transparenten Gegenstands **5** bestimmt werden soll, ist die Brennweite der Fokussiereinheit **33** derart gewählt, dass deren Brennpunkt auf der Gegenstandsvorderfläche **37** liegt. Die Brennweite der Fokussiereinheit **34** ist derart gewählt, dass deren Brennpunkt auf der Gegenstandsrückseite **39** liegt. Das optische Teilsystem **43** [ ▸Umlenkspiegel **35** - Fokussiereinheit **33** - Umlenkspiegel **36** ◂ ] ist parallel zum optischen Teilsystem **44** [▸Strahlteiler **31** - Linse **34** - Strahlteiler **32**◂], wie durch den Pfeil **41** angedeutet, verschiebbar. Der optische Weg der durch das Teilsystem **43** laufenden Strahlung ist durch eine Verschiebung in Pfeilrichtung **41** veränderbar. Da die Messstrahlung im bzw. auf dem Gegenstand **5** reflektiert wird, werden die optischen Teilsysteme **43** und **44** zweimal durchlaufen. Die optische Wegdifferenz (nur Hin- oder Rückweg) wird nun derart eingestellt, dass sie dem im Gegenstand **5** auszumessenden optischen Abstand, hier der optischen Gegenstandstiefe, ungefähr entspricht.

Für die nachfolgende Beschreibung des Messverfahrens wird die Wirkungsweise der λ/2- bzw. λ/4Platten **25** und **27** vorerst außer Acht gelassen. Der von der Strahlungsquelle **7** ausgesandte, linear polarisierte Quellenstrahl **9** trifft auf den Strahlteiler **11** und wird von diesem in gleiche Teile für den Referenz- und den Messarm **1** und **3** als Referenz- und Gegenstandsstrahl **13** und **15** aufgeteilt. (Eine Aufteilung in gleiche Teile ist jedoch nicht zwingend notwendig.) Die Strahlteiler **31** und **32** sind als polarisierende Strahlteiler ausgebildet. Der Strahlteiler **31** ist nun derart ausgebildet und orientiert, dass der durch den Strahlteiler **11** geteilte polarisierte Quellenstrahl **9** mit der nun halben Strahlungsintensität als Gegenstandsstrahl **15** mit einer Polarisationsrichtung unter 45° auf den Strahlteiler **31** trifft. Hier wird der Gegenstandsstrahl **15** in zwei Messstrahlen **45** und **46** aufgeteilt, deren Polarisationsebenen nun 90° gegeneinander gedreht liegen. Der unter 45° polarisierte Referenzstrahl **13** enthält somit diese beiden Polarisationsrichtungen. Immer wenn eine Interferenz detektierbar ist, ist die Länge des optischen Weges ▸ Strahlteiler **11** - Strahlteiler **31** - Fokussiereinheit **34** - Strahlteiler **32** - Gegenstandsrückseite **39** und wieder zurück◂ gleich lang wie derjenige Weg, den der am Spiegel **17** reflektierte Referenzstrahl **13** durchläuft, wobei die optische Weglänge im Referenzarm **1** im Hertzbereich mit der Weglängenvariationseinheit **17** veränderbar ist. Die Fokussiereinheit **34** kann bei gewissen Anwendungen weggelassen werden . Es kann z. B. für die Augenlängenmessung die Fokussierung des Messstrahls **46** von der Brechkraft des Auges übernommen werden. Eine Längenzuordnung ist über den jeweiligen Drehwinkel der Einheit **17** möglich. (Bei einem linear verschiebbaren Spiegel ist die Verschiebung am Translationsstand erkennbar.) Eine weitere Interferenz ist detektierbar, wenn analog zu den gerade gemachten Ausführungen der optische Weg [ ▸ Strahlteiler **11** - Strahlteiler **31** - Umlenkspiegel **35** - Fokussiereinheit **33** - Umlenkspiegel **36** - Strahlteiler **32** - Gegenstandsvorderseite **37** und wieder zurück◂ ] gleich lang wie der am Spiegel **17** reflektierte Referenzstrahl **13** ist. Entspricht die Wegdifferenz durch die Umwegeinheit **29** dem Abstand von Gegenstandsvorder- zur -rückseite **37** bzw. **39,** so fallen die Interferenzen bei ein- und derselben Ortslage des Spiegels **17** (Weglängenvariationseinheit) zusammen. Wenn nicht, dann ergeben sich gegenüber dem tatsächlichen Abstand (Dicke) nur geringfügig unterscheidende Ortslagen.

Die optische Weglängendifferenz der beiden optischen Wege in der Umwegeinheit **29** werden immer so eingestellt, wie sie einem zu erwartenden ungefähren Messergebnis entsprechen würden. D.h. durch die Messung wird somit immer nur die Abweichung vom zu erwartenden Messergebnis ermittelt. Da diese Abweichungen immer bedeutend kleiner sind, als wenn der gesamte Weg (Abstand, Dicke, ...) ausgemessen werden muss, kann mit einer bedeutend kleineren und damit rascheren Weglängenvariation im Referenzarm gearbeitet werden. D.h. zeitlich gesehen, fallen die beiden Interferenzen sehr schnell hintereinander an; sie können sogar gleichzeitig erfolgen. Da bei Abständen, Dickenmessungen, etc. beim Stand der Technik immer zwei zeitlich gestaffelte Messungen vorzunehmen waren, liegt nun bei der Erfindung das Messergebnis derart schnell vor, dass Ortsverschiebungen des auszumessenden Gegenstands die Messgenauigkeit nur unwesentlich beeinflussen. In der Umwegeinheit **29** sind die Strahlteiler **31** und **32,** die Umlenkspiegel **35** und **36** sowie die beiden Fokussiereinheiten **33** und **34** derart angeordnet, dass die beiden Fokuspunkte auf der Gegenstandsvorder- und der -rückseite **37** und **39** auf einer optischen Achse **40** liegen.

Der gerade geschilderte Vorteil ist bei einer Augenlängenmessung an den Augen von Kindern, welche in der Regel nur schwer still zu stellen sind, von großem Nutzen.

Ist eine Zuordnung der auftretenden Interferenzen zu den betreffenden reflektierenden Flächen gewünscht, so können anstelle des einen Photodetektors **24** zwei, jeder für eine Polarisationsrichtung, verwendet werden. Es wird dann die Strahlung der einen Polarisationsrichtung mittels eines polarisierenden Strahlteilers auf den einen Photodetektor und die Strahlung der anderen Polarisationsrichtung auf den anderen Photodetektor geleitet.

Die Strahlungsreflexion kann nun an der Gegenstandsvorder- sowie an der rückseite **37** bzw. **39** unterschiedlich hoch sein; auch können Reflexionen von Bereichen innerhalb eines Gegenstands, deren Abstand man bestimmen möchte oder sofern es sich um Schichten handelt, deren Dicke man ermitteln möchte, unterschiedlich sein. Um nun die reflektierte Intensität in einem gewissen Rahmen anpassen zu können, sind die $\lambda/2$- bzw. $\lambda/4$ Platten **25** und **27** im Quellenstrahl **9** und im Referenzstrahl **13** vorhanden. Man kann nun die jeweilige Platte derart verstellen, dass in dem Strahl, dessen Strahlung schwach reflektiert wird, mehr Intensität eingekoppelt wird.

Die Weglängenänderung im Referenzarm **1** beaufschlagt die Strahlungsfrequenz des Referenzstrahls **13** mit einer Dopplerfrequenz $f_{Doppler}$ gemäß der Beziehung

$$f_{Doppler} = \frac{2 \cdot f_0 \cdot v_{scan}}{c},$$

wobei **$f_0$** die Strahlungsfrequenz der Strahlungsquelle 7, **$v_{scan}$** die Weglängenänderungsgeschwindigkeit und **c** die Lichtgeschwindigkeit ist. (Mit der in der WO96/35100 beschriebenen Weglängenvariationseinheit ist die Dopplerfrequenz **$f_{Doppler}$** annähernd konstant.) Diese Dopplerfrequenz weist auch das mit dem Photodetektor **24** detektierte Interferenzsignal auf. Das vom Detektor **24** erhaltene elektrische Signal kann somit mit einem elektronischen Bandpassfilter von der restlichen detektierten Strahlung getrennt werden. Hierdurch verbessert sich das Signal-Rausch-Verhältnis erheblich.

Es müssen nun nicht alle Strahlverläufe, wie in **Figur 1** dargestellt, als freie "Raumstrahlen" verlaufen. Es können auch Fasern als Strahlungsleiter, wie in **Figur 2** dargestellt. verwendet werden. Analog zur Strahlungsquelle **7** wird in **Figur 2** eine Laserdiode **50**, eine Superlumineszenzdiode, LED oder eine andere breitbandige Lichtquelle verwendet, deren Strahlung über einen Strahlungsleiter **51** zu einem Faserkoppler **52** geführt wird. Im Faserkoppler **52** erfolgt eine Aufteilung der Strahlung der Laserdiode **50** in die beiden Strahlungsleiter **53a** und **53b,** wobei der Strahlungsleiter **53a** Bestandteil des Messarms **55** und der Strahlungsleiter **53b** Bestandteil des Referenzarms **56** ist.

Im Strahlungsleiter **53b** des Referenzarms **56** ist ein Faserpolarisationsregler **57** analog zur $\lambda/4$-Platte **27** integriert. An dem dem Faserkoppler **52** abgewandten Ende des Strahlungsleiters **53b** ist ein Kollimator **59** angeordnet, welcher die aus dem Leiterende austretende Strahlung in einen parallelen Freiraumstrahl **60** verwandelt. Die Intensität dieses Freiraumstrahls **60,** insbesondere im Verhältnis zur im Messarm **55** reflektierten Strahlung, kann anschließend an den Kollimator **59** mit einem Graufilter **61** (Dämpfung) und einer weiteren $\lambda/4$-Platte **63** verändert werden. Im Gegensatz zu einem translatorisch verschiebbaren Referenzspiegel **17** ist in **Figur 2** als Wegvariationseinheit ein rotierender transparenter Würfel **65** mit einem Spiegel **66,** der die auf ihn fallende Strahlung in sich selbst reflektiert, verwendet (WO 96/35100).

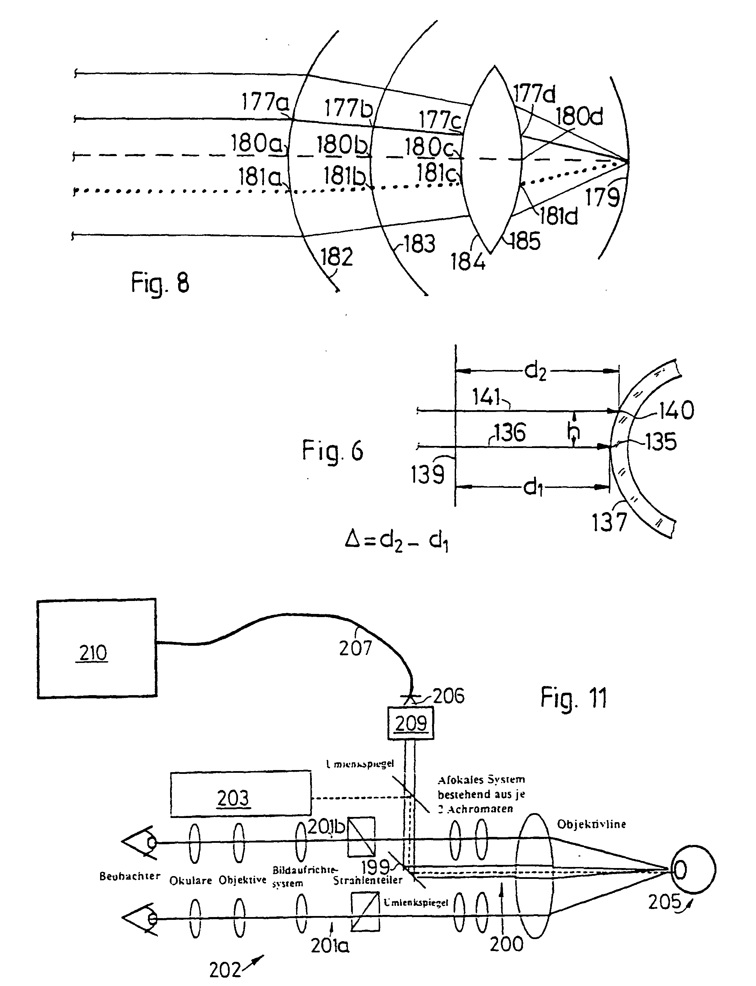

Im Messarm **55** ist am dem Faserkoppler **52** angewandten Ende des Strahlungsleiters **53a** ein Kollimator **67** angeordnet, welcher die aus dem Leiterende austretende Strahlung in einen parallelen Freiraumstrahl **69** umformt. Der Freiraumstrahl **69** wird, wie bereits oben ausgeführt, mit einer zur Umwegeinheit **29** analog ausgebildeten Umwegeinheit **70** in zwei unterschiedliche Laufzeiten aufweisende Messstrahlen aufgespalten und wieder mit einer gemeinsamen optischen Achse als gemeinsame Strahlkonfiguration **71** überlagert. Die die beiden Messstrahlen aufweisende Strahlkonfiguration **71** durchläuft ein Linsensystem **72** zur Strahldurchmesserminimierung auf den Polygonflächen eines rotierenden Polygonspiegels **73**. In Folge der Rotation des Polygonspiegels **73** wird die Strahlkonfiguration **71** über das Auge **75** in der dort gezeigten Pfeilrichtung **76** abgelenkt. Es kann somit ein Augenlängenprofil aufgenommen werden. Mit den abgespeicherten Werten einer Oberfläche kann mit Bezug auf eine Referenzebene, wie unten beschrieben (siehe **Figur 6**), deren Oberflächenprofil ermittelt werden. Mit dem Linsensystem **77** und einem Linsensystem **78** im Umweg wird eine Anpassung der Fokuspunkte der beiden Messstrahlen derart vorgenommen, dass der den Umweg aufweisende Strahl infolge der Linsensysteme **67** und **78** sowie der Linsensysteme **72** und **77** auf die Hornhaut **79** des Auges **75** und der den Umweg nicht aufweisende Messstrahl auf den Augenhintergrund fokussiert ist. Hiermit ist dann eine Augenlängenmessung vomehmbar.

Die im Messarm **55** rückreflektierte Strahlung vom Auge **75** wird dann mit der im Referenzarm **56** rückreflektierten und mit einer Dopplerfrequenz überlagerten Strahlung im Faserkoppler **52** interferierend überlagert und vom über einen Strahlungsleiter **80** mit dem Faserkoppler **52** verbundenen Detektor **82** analog der obigen Ausführung detektiert.

Anstelle nur eines einzigen Polygonspiegels können selbstverständlich auch zwei verwendet werden, um ein zweidimensionales Abfahren (Abscannen) zu erreichen. Anstelle eines regulären Polygonspiegels kann auch ein Polygonspiegel verwendet werden, dessen Spiegelflächen einen sich sukzessive verändernden Neigungswinkel gegenüber der Rotationsachse aufweisen, um ein zeilenartiges Abscannen zu erreichen. Die Rotationsgeschwindigkeiten zwischen dem "Würfel" der Wegvariationseinheit und dem Polygonspiegel müssen nicht koordiniert sein; man muss für die computerisierte Auswertung nur die jeweiligen Positionen wissen.

Anstelle eines Polygonspiegels können auch sogenannte Galvanometerspiegel-, elektro-optische und akusto-optische Ablenker verwendet werden. Auch kann ein elektro-magnetisch bewegter Spiegel verwendet werden, der auf einer Kugeloberfläche derart gelagert ist, dass eine zweidimensionale Ablenkung möglich ist. Vorteil dieser Anordnung gegenüber einem Polygonspiegel ist ein Drehpunkt nahe der Spiegeloberfläche.

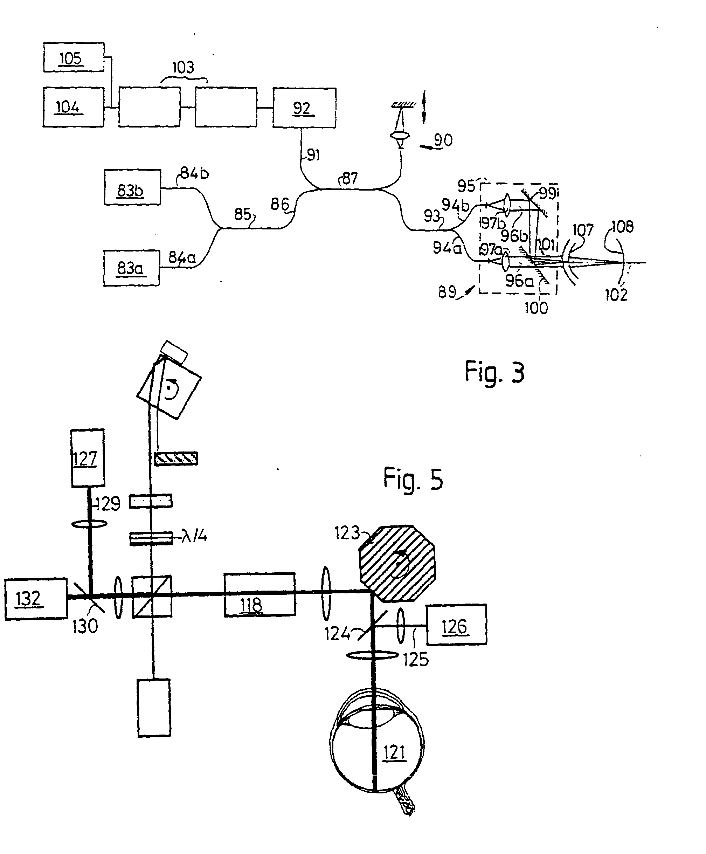

Figur 3 zeigt zu den in den **Figuren 1** und **2** dargestellten optischen Anordnungen eine weitere Ausführungsvariante der Erfindung, bei der zwei Strahlungsquellen **83a** und **83b** mit unterschiedlichen Zentrumswellenlängen verwendet werden. Die Spektren der verwendeten Lichtquellen sollten sich möglichst wenig überlappen. Analog zum optischen Aufbau in **Figur 2** wird auch hier mit Strahlungsleitern gearbeitet. Die von den beiden Strahlungsquellen **83a** und **83b** ausgehenden Strahlungsleiter **84a** und **84b** werden in einem ersten Faserkoppler **85** in einen Strahlungsleiter **86** eingekoppelt, der mit einem weiteren Faserkoppler **87** verbunden ist. Vom Faserkoppler **87** geht ein Messarm **89** und ein Referenzarm **90** weg sowie ein Strahlungsleiter **91** zu einem Detektor **92**. Im Gegensatz zu den bereits oben beschriebenen Ausführungsvarianten hat hier der Messarm einen weiteren Faserkoppler **93**, der die von den beiden Strahlungsquellen **83a** und **83b** kommenden Strahlungen mit unterschiedlicher Zentrumswellenlänge wieder nach den Zentrumswellenlängen in zwei Strahlungsleiter **94a** und **94b** auftrennt. Die Enden der beiden Strahlungsleiter **94a** und **94b** enden in einer Umwegeinheit **95**. Die Umwegeinheit **95** hat für die aus den Enden der Strahlungsleiter **94a** und **94b** austretenden Freiraumstrahlungen **96a** und **96b** je ein Linsensystem **97a** und **97b**. Die Freiraumstrahlung **96b** wird dann über einen Umlenkspiegel **99** mit einem wellenlängenselektiven Strahlteiler (-vereiniger) **100** zu einer eine einzige optische Achse **102** aufweisenden Strahlkonfiguration **101** vereinigt. Die Linsensysteme **97a** und **97b** sind nun gerade derart ausgewählt, dass eine Fokussierung der betreffenden Freiraumstrahlung **96a** bzw. **96b** auf die betreffende Trennfläche, hier beispielsweise **107** und **108**, zur Dicken- bzw. Abstandbestimmung erfolgt. Eine Verstellung des Umwegs ist in der hier beschriebenen Ausführungsvariante einfach dadurch zu erzielen, dass z. B. lediglich der Umlenkspiegel **99** samt dem Linsensystem **97b** und dem Ende des Strahlteilers **94b** bezüglich des Strahlteilers **100** gemäß der Darstellung in **Fi-**

gur 3 nach oben verschoben wird. Dies ist einfach durchführbar, da die Strahlungsleiter **94a** und **94b** flexibel sind.

Die mit dem Photodetektor **92** erhaltenen elektrischen "Interferenzsignale" werden dann mit einer Auswerteelektronik **103** weiter verarbeitet, eventuell auf einem Bildschirm **104** dargestellt und können über ein Datenübertragungsnetz **105** zur weiteren Ausarbeitung und Abspeicherung weitergegeben werden.

Zur Messung an zwei in axialer Richtung getrennt liegenden Bereichen in einem Gegenstand ist die oben beschriebene unterschiedliche Polarisation sowie auch die beiden unterschiedlichen Wellenlängen im umgelenkten sowie im direkten Strahl des Messarms nicht notwendig. Auf die λ/4- und λ/2-Platten kann dann ebenfalls verzichtet werden. Die polarisationssensitiven bzw. wellenlängensensitiven Strahlteiler bzw. Faserkoppler können dann durch normale physikalische Strahlteiler ersetzt werden. Ein Vorteil eines Arbeitens mit unterschiedlichen Strahleigenschaften [unterschiedliche Frequenz, unterschiedliche Polarisationsrichtung] liegt in einer Empfindlichkeitserhöhung um den Faktor 8. Wird nämlich ununterscheidbare Strahlung verwendet, so geht auf dem Hinweg die Hälfte der Strahlung beim Strahlteiler **32** bzw. **100** und auf dem Rückweg geht zusätzlich zweimal die Hälfte verloren. Nämlich zuerst beim Strahlteiler **32** bzw. **100** sowohl für die von der Gegenstandsvorderseite, als auch für die von der Gegenstandsrückseite reflektierten Strahlung. Durch den "falschen" Umweg ist diese Strahlung für die interferometrische Detektion wegen der "falschen" Weglänge "verloren". Ferner geht noch einmal die Hälfte der Strahlung beim Strahlteiler **31** oder beim Faserkoppler **93** sowohl für die von der Gegenstandsvorderseite als auch für die von der Gegenstandsrückseite reflektierte Strahlung verloren. Ist eine ausreichende Strahlungsintensität vorhanden und erträgt der Gegenstand diese hohe Intensität, so ist dies eine bevorzugte Ausführungsvariante, da hier die zu verwendenden optischen Komponenten preisgünstiger sind.

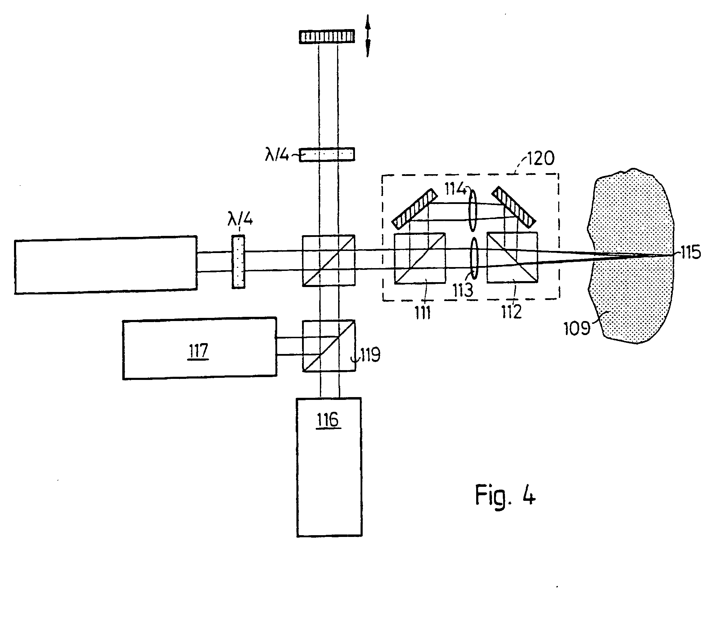

Soll die Phasenverzögerung in einem doppelbrechenden Material eines Gegenstands **109** gemessen werden, so wird eine Vorrichtung mit einem optischen Aufbau gemäß **Figur 4** verwendet. Der in **Figur 4** dargestellte Aufbau entspricht bis auf die nachfolgend angeführten Unterschiede demjenigen der **Figur 1**.

Die Strahlteiler **31** und **32** des Aufbaus von **Figur 1** sind in **Figur 4** die analogen Strahlteiler **111** und **112**. Ferner sind die den Linsensystemen **33** und **34** entsprechenden Linsensysteme **113** und **114** derart ausgebildet, dass die durch sie erzeugten Brennpunkte in einem Bereich, hier z.B. auf der Probenrückseite, zusammenfallen; es soll ja eine Phasenverzögerung zweier Strahlungen mit zueinander senkrecht stehender Polarisationsebenen ermittelt werden. Anstelle eines einzigen Photodetektors in **Figur 1** sind in **Figur 4** jetzt für jede Polarisationsrichtung ein Photodetektor **116** und **117** vorhanden, wobei die auf sie fallende, von der Probenrückseite **115** reflektierte Strahlung, der die Referenzstrahlung interferierend überlagert wird, durch einen polarisierenden Strahlteiler **119** aufgeteilt wird.

Die von der Probenrückseite **115** reflektierte p-polarisierte Strahlung wird mit vemachlässigbaren Verlusten auf dem kurzen Weg durch die Umwegeinheit **120** über den Strahlteiler **119** auf den Photodetektor **116** gestrahlt. Die ebenfalls von der Probenrückseite **115** reflektierte s-polarisierte Strahlung - die Polarisationsebene der s-Strahlung liegt senkrecht zu derjenigen der p-Strahlung - wird ebenfalls mit vernachlässigbaren Verlusten auf dem langen Weg durch die Umwegeinheit **120** über den Strahlteiler **119** auf den Photodetektor **117** gestrahlt. Da ausschließlich von der Probenrückseite **115** reflektierte Strahlung detektiert wird, ist die Umwegeinheit **120** derart einzustellen, dass die optischen Wegunterschiede zwischen der s- und p-Strahlung möglichst klein sind. Aus den durch die Photodetektoren **116** und **117** gemessenen Intensitäten der betreffenden reflektierten Intensitäten kann dann auf die Phasenverzögerung des doppelbrechenden Materials geschlossen werden.

Soll neben einer der oben beschriebenen Messungen zusätzlich eine Betrachtung vorgenommen werden, so kann eine Vorrichtung mit einem optischen Aufbau, wie in **Figur 5** gezeigt, beispielsweise verwendet werden. Die optische Anordnung der **Figur 5** entspricht im wesentlichen den in den **Figuren 1** bis **4** gezeigten Anordnungen. Eine den Umwegeinheiten **29; 70** und **120** entsprechende Umwegeinheit **118** ist lediglich angedeutet. Die Ausführungsvariante gemäß **Figur 5** weist jedoch die nachfolgenden Unterschiede auf. Zur Beleuchtung des Gegenstands, hier eines Auges **121**, ist nach einem, in **Figur 2** bereits beschriebenen Polygonspiegel **123** ein Strahlteiler **124** vorhanden, über den die Beleuchtungsstrahlung **125** einer Strahlungsquelle **126** eingekoppelt wird. Mit einer Kamera **127** kann der auszumessende Bereich betrachtet und auf einem nicht dargestellten Monitor wiedergegeben werden. Der Beobachtungsstrahl **129** wird mit einem Strahlteiler **130** auf die Kamera **127** gelenkt. Erscheint der Beleuchtungsstrahl auf dem Monitor **128** in einem bestimmten örtlichen Toleranzbereich, so ist das Auge ausgerichtet und die Messung kann ausgelöst werden.

Sollen sehr kleine Dicken oder Abstände ermittelt werden, kann es vorkommen, dass der in den **Figuren 1** bis **5** gezeigte Umweg in der dort skizzierten Umwegeinheit infolge der geometrischen Abmessungen von zu verwendenden optischen Komponenten zu lang ist. In diesem Fall kann analog zu dem dort jeweils gezeigten großen optischen Weg auch der kurze optische Weg verlängert werden, in dem auch hier zwei verschiebbare Umlenkspiegel in den Strahlengang eingesetzt werden. Mit einer derartigen Umweganordnung können dann beliebig kurzer und langer Umweg vertauscht werden.

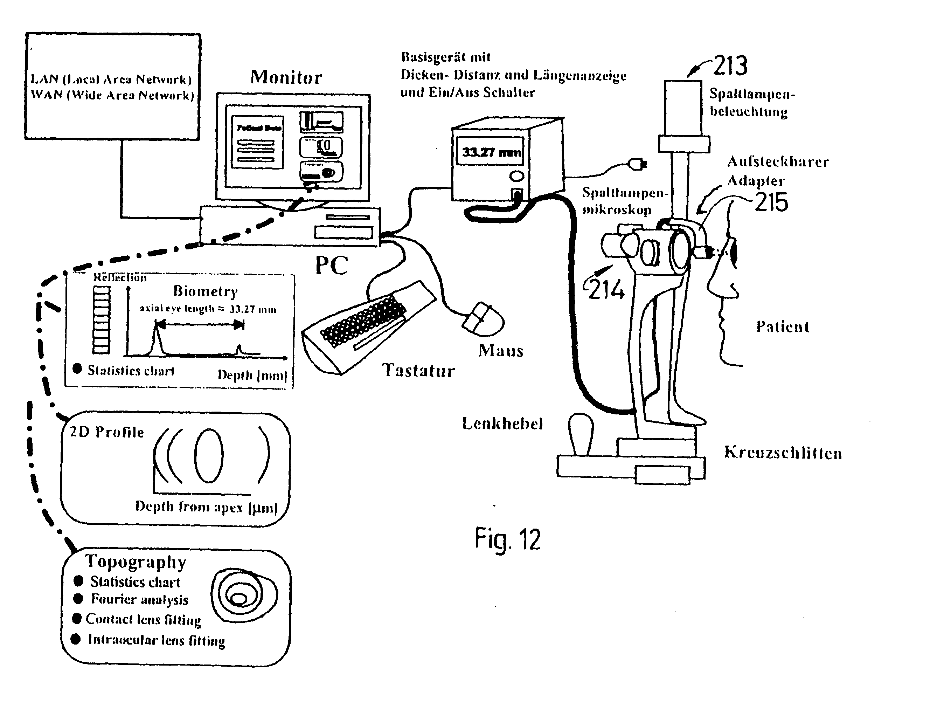

Die oben beschriebene erfindungsgemäße Vorrichtung kann nebst ihren Ausführungsvarianten zusammen mit bereits bestehenden Geräten verwendet werden. Diese Vorrichtung kann man z. B. in ein Spaltlampengerät für die Augenuntersuchung einbauen oder mit diesem kombinieren. Es kann dann der Messstrahl als Freiraumstrahl entweder via Strahlteiler in den Beleuchtungsstrahlengang, beim Mikroskop ebenfalls via Strahlteiler in einen Bebachtungsstrahlengang oder beim Mikroskopobjektiv oder mit einem Umlenkspiegel **199** in einen Mittelkanal **200** eines Stereomikroskops **202** eines Spaltlampengeräts, wie in **Figur 11** gezeigt, eingekoppelt werden. Der Mittelkanal **200** liegt zwischen den beiden Strahlengängen **201a** und **201 b** des Stereomikroskops **202**. In **Figur 11** ist zudem noch eine Fixationslichtquelle **203** gezeigt. Durch eine Betrachtung der Fixationsquelle **203** richtet der Patient sein Auge **205** auf einen vorgegebenen Ort aus und hält es meistens auch auf diesen unbeweglich fixiert. Der Messstrahl **206** tritt (analog einer Vorrichtungsanordnung wie sie **Figur 2** zeigt) aus einer Faser **207** aus und durchläuft eine analog zur Umwegeinheit **29, 70, 89** oder **120** ausgebildete, nicht explizit dargestellte Umwegeinheit **209** mit einem optionalen transversalen Scanner. Die restlichen Elemente der erfindungsgemäßen Vorrichtung sind in einem kompakten Basisgerät **210** eingebaut. Bei der Wahl der in der Umwegeinheit **209** verwendeten Linsen muss lediglich die Linsenwirkung der Objektivlinse des Spaltlampenmikroskops **202** mit berücksichtigt werden.

Durch die Verstellung des Spaltlampengeräts in den drei räumlichen Koordinaten, bevorzugt mit einem sogenannten Lenkhebel, wird dann auch der Messstrahl entsprechend verstellt. Anstatt das ganze Spaltlampengerät zusammen mit dem Messstrahl zu verstellen, können auch beide unabhängig voneinander verstellt werden. Wie bereits oben angedeutet, verwendet man bei einer Verstellung nur des Messstrahls bevorzugt eine "faseroptische" Ausführung analog zur Darstellung in **Figur 3**.

Bei einer Kombination mit einem mit Placidoringen ausgerüsteten Videokeratographen erfolgt eine Einkopplung des Messstrahls in Richtung der Beleuchtungsachse des Videokeratographen mit Hilfe eines kleinen Strahlteilers.

Anstatt den Messstrahlengang, wie oben beschrieben in ein Stereomikroskop zu integrieren, kann er auch bei einem Spaltlampengerät **213** über einen auf das Mikroskop **214** aufsteckbaren Adapter **215** zugeführt werden, wie in **Figur 12** skizziert ist. Der Adapter **215** beinhaltet die Funktionen der Umwegeinheit **29, 70, 95** oder **120.**

Aufgrund der obigen Ausführung kann die Vorrichtung gemäß den **Figuren 1** bis **5** mit diversen Geräten kombiniert werden. Sie kann jedoch auch, da der Messvorgang äußerst rasch erfolgt, als Handgerät ausgebildet werden, welches dann lediglich vor einen auszumessenden Gegenstand gehalten wird. Bei einer Ausbildung als Handgerät bzw. als modifiziertes Spaltlampengerät **(Figur 12)** wird man bevorzugt die in **Figur**

**3** gezeigte Ausführungsvariante mit Faserleitern verwenden, da dieses eine große Bewegungsfreiheit ergibt. In diesem Fall sind vorzugsweise die bewegbaren Fasern als polarisationserhaltende Fasern ausgebildet. Es kann beispielsweise auch eine Kombination mit einem sogenannten Kontaktglas erfolgen, welches in der Hand gehalten an die Homhautoberfläche des menschlichen Auges angelegt wird.

Um diese Vorrichtung möglichst universell verwendbar auszuführen, müsste nun, da die Länge des Messarmes (Abstand der Umwegeinheit zum auszumessenden Gegenstand) stark variieren kann, das Weglängenvariationselement einen großen Variationsweg durchfahren können. Interferenzen ergeben sich nämlich nur, wenn Referenzweg und entsprechender Messweg gleich lang sind. Diese Differenzen muss das Weglängenvariationselement ausgleichen können. Ein großer Variationsweg macht einerseits die Weglängenvariationseinheit teuer in ihrer Ausführung und verlängert zudem die Messzeit.

Dieser Nachteil kann umgangen werden, wenn versucht wird, den Messweg innerhalb kleiner Toleranzwege zu halten. Zwei Strahlen der Strahlungsquelle können nun derart angeordnet werden, dass sie sich erst im Messgebiet auf dem auszumessenden Gegenstand treffen, wenn dieser sich innerhalb eines vorgegebenen Abstands befindet. Man wird hierzu die zwei Justierstrahlen (Pilotlaser) gegen die optische Achse geneigt einstrahlen. Zur Justage werden dann zwei Punkte auf einer streuenden oder reflektierenden Oberfläche des Messobjekts gesehen, welche erst bei richtiger Entfernungseinstellung zu einem Punkt verschmelzen.

Bei Verwendung eines Betrachtungsmikroskops kann der Umweg in der Umwegeinheit optimal dadurch eingestellt werden, dass der Strahldurchmesser jedes Messstrahls minimal auf den betreffenden Bereich eingestellt wird. Dieser Vorgang kann auch automatisiert werden; z.B. mit einer CCD-Kamera, einer Auswerte- und Steuereinheit und einem Aktuator. Die Steuereinheit, mit der der Aktuator gesteuert wird. regelt die Distanz zwischen der Umwegeinheit und dem Messobjekt (Gegenstand).

Anstatt nur jeweils einen Abstand bzw. eine Dicke auszumessen, können auch mehrere ausgemessen werden, indem anstelle nur eines Umwegs mehrere vorgesehen werden. Weitere Umwege können erhalten werden, indem jeweils ein Umlenkspiegel durch einen Strahlteiler mit entsprechenden Eigenschaften ersetzt wird und dann wieder ein Umlenkspiegelpaar oder nur ein einziger Umlenkspiegel bei der Faseranordnung gemäß **Figur 3** verwendet wird.

In den vorgängig beschriebenen **Figuren 1** bis **5** werden Messungen ausgeführt, bei denen eine Dicke ermittelt wird. Hierzu wird der erste Messstrahl auf einen ersten Punkt fokussiert und der zweite Messstrahl, in der Regel derjenige, welcher den Umweg nicht durchlaufen hat, auf einen zweiten, nach dem ersten Punkt liegenden Punkt fokussiert. Erster und zweiter Punkt liegen hier auf einer optischen Achse. Man kann nun die erfindungsgemäße Vorrichtung auch derart aus- und umbilden, dass die Fokuspunkte der beiden Messstrahlen nebeneinander liegen. Werden die Messstrahlen seitlich nebeneinander gelegt, so kann bei einer Oberfläche, welche wenigstens einen Mindestreflexionsfaktor von $10^{-4}\%$ hat, ein Oberflächenprofil bestimmt werden. Es wird hierzu, wie in **Figur 6** angedeutet ist, der Abstand $d_1$ eines ersten reflektierenden Ortes **135** des ersten Messstrahls **136** auf der Oberfläche **137** von einem Referenzpunkt bzw. einer Referenzebene **139** und der Abstand $d_2$ des zweiten reflektierenden Ortes **140** des zweiten Messstrahls **141** von der Referenzebene **139** ermittelt. Beide Messwerte werden in einer Speichereinheit **143** abgespeichert. Die Abstandsdifferenz $d_1$ und $d_2$ der beiden Messstrahlen **136** und **141** von der Referenzebene **139** in Relation zu deren gegenseitigen Abstand **h** ergibt dann zwei Oberflächenkoordinaten. Aus diesen beiden Koordinaten kann dann auf den Oberflächenverlauf mit Näherungsverfahren geschlossen werden, sofern die Art der Oberfläche bekannt ist. Die Art der Oberfläche ist beim menschlichen Auge bekannt. Werden mehrere Messstrahlen verwendet oder mehrere Messungen mit seitlich versetzten Messstrahlen vorgenommen, kann die Oberfläche genauer ermittelt werden.

In der Ophthalmologie spielt bei der Anpassung von intraokulären Linsen in der Katarakt-Behandlung nicht nur die Augenlänge und die Vorderkammertiefe eine wesentliche Rolle, sondern auch das Krümmungsprofil der Hornhaut, vor allem in deren Zentrum. Alle diese Werte können mit der erfindungsgemäßen Vorrichtung bestimmt werden.

Zur Ermittlung des Profils genügen im Minimalfall zwei bestimmte Krümmungsradien der zentralen Hornhaut, nämlich ein Krümmungsradius in horizontaler und einer in vertikaler Richtung. Sind diese beiden Radien verschieden, spricht man von einem (zentralen) Astigmatismus. Die Krümmungsradien können mit Hilfe bekannter geometrischer Algorithmen ermittelt werden, wenn pro zu bestimmenden Kreisbogen, wie bereits oben ausgeführt, ausgehend von einer Referenzebene (hier **139**) der Abstand unter einem vorgegebenen Winkel (hier der normale Abstand $d_1$ und $d_2$) und der Abstand (hier **h**) der Kurvenpunkte (hier **135** und **140**) von einander bekannt sind. Die Abstände $d_1$ und $d_2$ sind aus dem augenblicklichen Ort des reflektierenden Spiegels in der betreffenden Weglängenvariationseinheit bei auftretender Interfenzerscheinung ermittelbar. Als Referenzwert wird eine vorgegebene Spiegelstellung verwendet. Wird eine Weglängenvariationseinheit mit einem rotierenden Würfel (beispielsweise wie in der WO 96/35100 beschrieben) verwendet, wird man bevorzugt dessen Nullgrad-Position als Referenz verwenden, bei der der einfallende Strahl senkrecht auf die erste Würfeloberfläche trifft. Anstelle vom minimal drei Messstrahlen zur Ermittlung der beiden zentralen Krümmungsradien können auch mehrere Messstrahlen verwendet werden, um eine exaktere Krümmungsradienausmessung vorzunehmen. Auch kann gleichzeitig Dicken- und Radienmessung vorgenommen werden, wie unten ausgeführt wird.

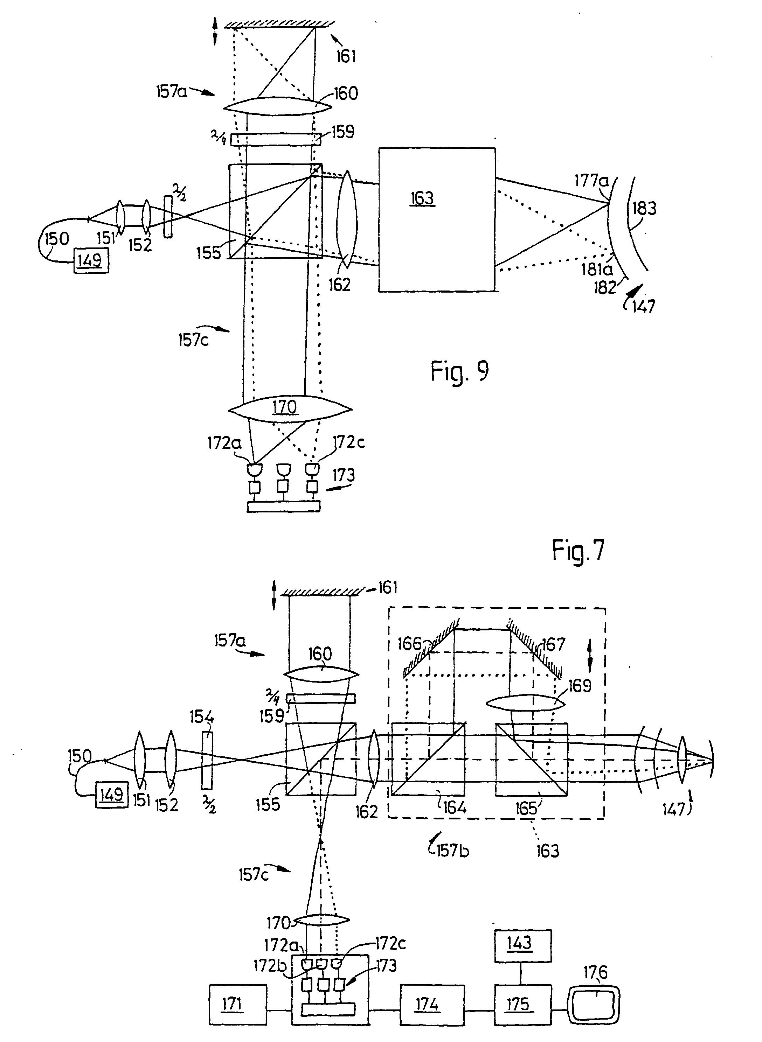

Die in **Figur 7** an Hand eines optischen Blockschaltbildes beschriebene Vorrichtung dient zur Ermittlung eines Oberflächenprofils sowie diverser Dicken bei einem transparenten bzw. diffusiven Gegenstand, hier einem menschlichen Auge **147**. Der in **Figur 7** schematisch dargestellte optische Aufbau ähnelt in weiten Teilen demjenigen der **Figur 1**. Auch hier ist eine Strahlungsquelle **149** vorhanden, welche beispielsweise eine superlumineszente Diode sein kann. Die Strahlung der Strahlungsquelle **149** wird hier jedoch über einen Strahlungsleiter **150** geführt, der eine Ortsunabhängigkeit von Strahlungsquelle **149** und der Mess- und Auswerteeinheit ermöglicht. Die aus den Strahlungsleiter **150** austretende Strahlung wird mit einer Linse **151** kollimiert und mit einer zweiten, nachfolgenden Linse **152** fokussiert. Zwischen dem Fokuspunkt **153** und der Linse **152** ist zur "Drehung" der Polariationsrichtung der Strahlung eine λ/2-Platte **154** angeordnet. Anschließend folgt ein Strahlteiler **155,** mit dem die Strahlung in den Messarm **157b** und den Referenzarm **157a** aufgeteilt wird. Der Referenzarm **157a** hat auf den Strahlteiler **155** folgend eine λ/4-Platte **159,** gefolgt von einer Linse **160,** mit der die vom Strahlteiler **155** kommende Strahlung kollimiert wird. Als Weglängenvariationseinheit **161** ist schematisch lediglich ein linear verstellbarer Spiegel angedeutet; es können jedoch auch anders ausgebildete Einheiten, wie beispielsweise der rotierende Würfel verwendet werden.

Im Messarm **157b** folgt auf den Strahlteiler **155** eine Kollimationslinse **162** und dann eine analog zur Umwegeinheit **29** aufgebaute Umwegeinheit **163,** mit zwei polarisierenden bzw. wellenlängensensitiven Strahlteilern **164** und **165,** zwei Umlenkspiegeln **166** und **167** und einer Fokussierlinse **169.**

Die vom Auge rückreflektierte Strahlung wird mit der vom Referenzarm **157a** kommenden Referenzstrahlung überlagert und im Detektorarm **157c** über eine Linse **170** auf ein Detektionsarray **171** geführt, wobei der Einfachheithalber nur eine lineare und keine zwei dimensionale Darstellung von lediglich drei nebeneinander angeordneten Detektoren **172a, 172b** und **173c** vorgenommen worden ist. Jedem Detektor **172a, 172b** und **173c** ist eine elektronische Schaltung **173** beispielsweise mit einem Verstärker, Dopplerfrequenzfilter, Gleichrichter und Tiefpassfilter nachgeschaltet. Die detektierten Messignale werden dann über einen Analog-Digital-Wandler **174** und einen Computer **175** mit der Speichereinheit **143** verarbeitet und auf einem Bildschirm **176** dargestellt.

Mit der in **Figur 7** schematisch dargestellten Vorrichtung kann die Augenlänge, die Korneadicke, die Vorderkammertiefe, die Linsendicke, die Glaskörpertiefe und die Retinaschichtdicke gleichzeitig an verschiedenen Stellen gemessen werden. Da an verschiedenen Stellen gemessen werden kann, können auch Oberflächenprofile rechnerisch ermittelt werden. Um dies zu verdeutlichen sind drei seitlich versetzte Strahlengänge ausgezogen, gestrichelt und punktiert dargestellt, welche auf die Detektoren **172a, 172b** und **172c** geführt werden. Der gestrichelte Strahl kommt, wie der besseren Übersichtlichkeit wegen vergrößert in **Figur 8** dargestellt, von den Orten **177a, 177b, 177c, 177d** und der Retina **179**. Je nach Stellung der Spiegel **166** und **167** bzw. des Spiegels in der Wegvariationseinheit wird dann vom Detektor **172a** ein Interferenzsignal registriert. Für die Orte **180a** bis **180d** und **181a** bis **181d** gilt analoges. Mit Hilfe eines Detektorarrays bestehend aus m x n Photodetektoren ist es möglich m x n Orte auf oder im Auge **147** z. B. auf der Korneavorderfläche **182,** der Korneahinterfläche **183**, der Augenlinsenvorder bzw. -hinterfläche **184** und **185** gleichzeitig zu messen und auszuwerten. Nach einer gewissen Zeitdauer, welche abhängig ist von der Bewegungsgeschwindigkeit des Spiegels in der Wegvariationseinheit **161,** werden dann die mit "**b**", dann mit "**c**", mit "**d**" gekennzeichneten Orte detektiert und ausgewertet.

Die Linsen **160** und **162** können je nach Verwendung als ein- oder zweidimensionales Linsenarray ausgebildet sein.

Zum besseren Verständnis des Messvorganges sind in **Figur 9** zusätzlich die "Strahlenbegrenzungen" zum und vom Ort **177a** ausgezogen sowie zum und vom Ort **181a** punktiert im Referenz-, Mess- und Detektorarm **157a, 157b** und **157c** dargestellt. Die ausgezogenen bzw. punktierten Linien zeigen die zwei Randstrahlen im Referenz-, Mess- und Detektorarm **157a, 157b** und **157c,** welche die Messung der Raumkoordinate des Ortes **177a** bzw. **181a** ermöglichen, d. h. mit diesen Strahlen interferieren.

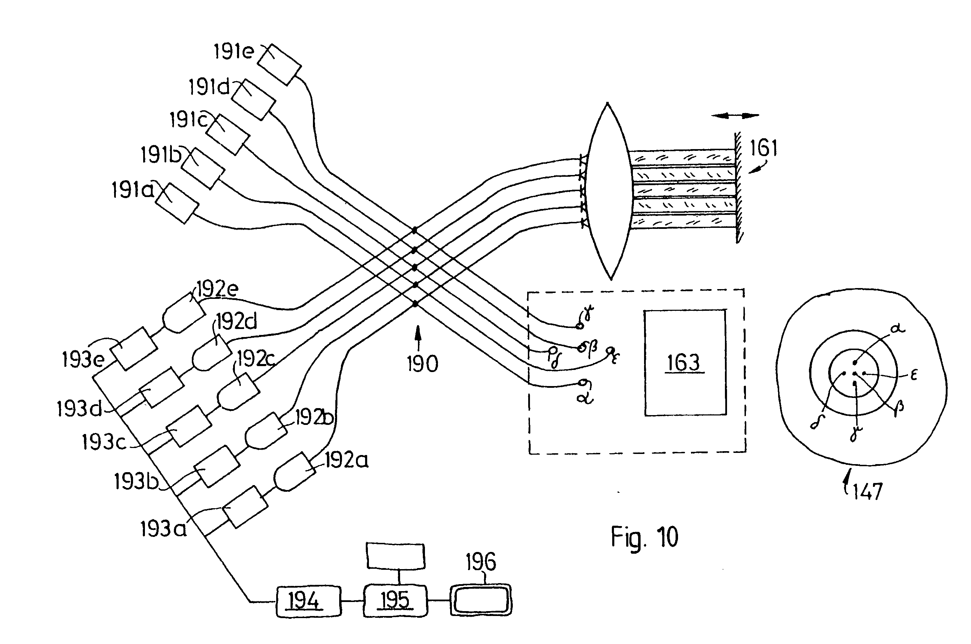

In **Figur 10** ist eine als faseroptisch-paralleles, kurzkohärentes Reflektormeter zu bezeichnende Vorrichtung skizziert. Diese Ausführungsvariante der Erfindung erlaubt beispielsweise die simultane Messung von vier zentralen Krümmungsradien der Hornhautvorderfläche in horizontaler (links und rechts) und vertikaler (oben und unten) Richtung. Zusätzlich ist auch die simultane Messung von vier zentralen Krümmungsradien der Hornhautrückseite möglich. Diese Vorrichtung hat fünf 2 x 2 Singelmodefaserkoppler **190,** fünf Strahlungsquellen **191a** bis **191e,** fünf Detektore **192a** bis **192e** mit zugehörender Schaltung **193a** bis **193e,** Analog-Digital-Wandler **194,** Computer **195** und Anzeige **196.** Die restlichen Elemente und Einheiten (insbesondere **161** und **163**) entsprechen denjenigen der **Figur 7**.

Die Eichung der oben beschriebenen Vorrichtungen kann dadurch erfolgen, dass in den Referenzarm (z.B. **1** oder **157a**) die Strahlung einer hochkohärenten Strahlungsquelle (z. B. ein distributed feedback Laser) mit einem (nicht dargestellten) Strahlteiler eingekoppelt wird. Die eingekoppelte Strahlung interferiert dann mit einem Strahlungsteil, der an einem beliebigen Ort zwischen diesem Strahlteiler und dem Weglängenvariator an einem feststehenden Reflektor reflektiert wird. Die Kohärenz der hochkohärenten Strahlungsquelle ist größer als die Wegvariationslänge des Variators. Über den Detektoren (oder auf einem separat hierfür eingerichteten Detektor) läuft dann ein Interferenzstreifenmuster. Der Abstand jeweils zweier Interferenzstreifen entspricht dann jeweils einer halben Wellenlänge. Durch ein (automatisches) Auszählen dieser Streifen ist eine Wegeichung des Weglängenvariators möglich. Da die hochkohärente Strahlung nicht auf das Auge des Patienten gelangen kann, kann deren Strahlungsleistung relativ hoch sein, so dass diese Detektion unkritisch ist. Die Wellenlänge der hochkohärenten Strahlung kann (muss es aber nicht) die gleiche Wellenlänge wie die für die Augenmessung verwendete kurzkohärente Strahlung haben.

Die mit den oben beschriebenen erfindungsgemäßen Vorrichtungen ermittelten Dicken der Hornhaut können vorzugsweise in eine Beratung von Patienten einfließen, bei denen eine refraktive Chirurgie mit LASIK (Laser-assisted in situ Keratomileusis) vorgenommen werden soll, in dem eine individuelle Berechnung eines Unterschieds zur kritischen Hornhautdicke mit Blick auf die aktuelle Hornhautdicke erfolgt. Hierzu wird man vorzugsweise die nachfolgenden neuen Schritte vornehmen:

1. Es wird eine präoperative zentrale Hornhautdicke $d_z$ mit einer der Vorrichtungen, wie sie die **Figuren 1** bis **5, 7** oder **10** zeigen, ermittelt.

2. Von der ermittelten Hornhautdicke $d_z$ wird die bei LASIK übliche mittlere Flapdikke $d_f$ von typischerweise 160 μm (einstellbar) abgezogen.

3. Es wird ein (maximal möglicher) Pupillendurchmesser ermittelt, während dem das Auge typischen nächtlichen Lichtintensitätsbedingungen ausgesetzt ist. Der "nächtliche Pupillendurchmesser" kann durch Abdunkeln des Untersuchungsraums mit einer an die erfindungsgemäßen Vorrichtungen bzw. deren Ausführungsvarianten angeschlossenen TV-Kamera ausgemessen werden. Eine derartige Kamera kann z. B. im Detektorarm via Strahlteiler mit einem entsprechenden Linsensystem angedockt werden. Die Messung des Pupillendurchmessers ist optional. Für eine Beratung können auch Standardwerte verwendet werden.

4. Es wird nun ein optimaler Ablationsdurchmesser **S** auf der Hornhaut festgelegt, der größer sein muss als der nächtliche Pupillendurchmesser, um nach der Ablation Haloerscheinungen zu vermeiden.

5. Die mit LASIK zu erreichende Korrketur in Dioptrien ist aus vorgängien Messungen (z. B. durch eine Kenntnis der Brechkraft einer bereits verwendeten Brille oder Kontaktlinse, welche der Patient bereits besitzt) bekannt.

6. Gemäß der Formel $t_0 = - (S^2 D)/3$ wird die für die gewünschte Korrektur notwendige zentrale Abtragungstiefe $t_0$ [in Mikrometer] für die gewünschte Korrektur berechnet, wobei **S** der optimale Ablationsdurchmesser in Millimeter und **D** die gewünschte Dioptrienänderung infolge der Ablation ist.

7. Es wird jetzt die zentrale stromale Restdicke $d_s = d_z - d_f - t_0$ berechnet, welche nach der LASIK-Operation erhalten werden würde.

8. Es wird abgeklärt, ob die Restdicke $d_s$ über einer kritischen zentralen stromalen Restdicke $d_k$ liegt. Eine mögliche Definition für die kritische zentrale stromale Restdicke $d_k$ ist beispielsweise $d_k = a \cdot d_z - b$, wobei a = 0,58 und b = 30 μm als Standardwerte angenommen werden.

9. Ist nun $d_s$ größer als $d_k$ kann eine LASIK-Operation empfohlen werden.

Die oben angeführten Verarbeitungsschritten können selbstverständlich über einen Rechner automatisiert werden.

Das Vorgehen bei einer Hyperopiekorrektur erfolgt analog. Die Hornhautdicke muss dann jedoch peripher an der Stelle der maximalen Abtragung gemessen werden; die unter Punkt 6 angegebene Formel ist dann entsprechend zu ersetzen.

Den oben angeführten Dicken- und Profilmessungen am Auge kann eine Bestimmung dessen Brechkraftverteilung hinzugefügt werden. Um dies zu erreichen, wird die Linse **162** in **Figur 7** durch ein (nicht dargestelltes) Linsenarray mit p x q Linsen ersetzt. Hierdurch wird die von der Strahlungsquelle **149** kommende Strahlung in p x q getrennte (nicht dargestellte) Teilstrahlen auf das Auge abgebildet. Das Linsenarray ist auf das Auge zu- bzw. von diesem wegbewegbar. Es wird nun in eine derartige Position gebracht, dass eine Fokussierung wenigstens teilweise auf der Retina erfolgt. An einem Ort zwischen der Augenoberfläche und der Linse **170** wird nun ein weiterer Strahlteiler eingesetzt und die Retina mit einer TV-Kamera betrachtet. Falls nun die örtliche Verteilung der Lichtpunkte auf der Retina von der durch das Linsenarray erzeugten Punktverteilung abweicht, ist die Brechkraftverteilung oder die Abbildungseigenschaft des Auges nicht ideal, d.h. das Auge bildet eine auf die Kornea auftreffende ebene Wellenfront nicht optimal ab. Diese Abweichung (z. B. sphärische Aberretion, Koma usw.) kann dann auf einem Monitor dargestellt werden.

Bekannte Tonometer (Augendruckmessgeräte) haben den Nachteil, dass sie den intraokularen Druck nur indirekt messen können. Die Messung erfolgt beispielsweise über eine Kraft, welche notwendig ist, um eine Hornhautoberfläche auf einer vorgegebenen Fläche abzuplatten (Applationstonometer). Die "abplattende" Kraft ist aber abhängig von der Hornhautdicke und der Krümmung der Hornhaut. Die bekannten Tonometer gehen von einer standardisierten Hornhautnormaldicke und -normalkrümmung aus. Bei einer Abweichung der Hornhaut von den Standardwerten stimmt dann ein derart ermittelter Augeninnendruck nicht mit dem tatsächlichen überein. Je dicker oder je stärker gekrümmt die Hornhaut ist, desto stärker weicht der bekanntermaßen ermittelte Innendruck vom tatsächlichen nach oben ab. Das kann dazu führen, dass wegen vermeintlich zu hohem Augendruck Medikamente zur Augendrucksenkung verabreicht werden, welche nicht nötig bzw. sogar schädlich sind. Diese Fehlmessung bzw. Fehlinterpretation kann jedoch auch dazu führen, dass beispielsweise ein Glaukom erst verspätet diagnostiziert wird.

Er wird nun vorgeschlagen, die erfindungsgemäße Vorrichtung mit einem Tonometer zu kombinieren. Der mit einem bekannten Tonometer gemessene ("falsche") Augeninnendruck wird rechnerisch unter Verwendung der mit der erfindungsgemäßen Vorrichtung ermittelten Homhautkrümmung und der Hornhautdicke korrigiert. Die Korrektur kann durch eine Eingabe der Werte in einen Rechner erfolgen oder automatisch durch eine elektronische Verknüpfung der beiden Geräte.

Die erfindungsgemäßen Vorrichtungen, ihre Ausführungsvarianten sowie ihre Messgeräte können vemetzt werden, wodurch auch eine Aufbereitung und Abspeicherung von Daten auch an entfernten Orten vorgenommen und mit anderen Daten verglichen werden kann.

Die erfindungsgemäße Vorrichtung dient wie bereits oben teilweise erwähnt zur ophthalmologischen Messung

- der Korneadicke, des Korneadickenprofils, des Komeavorder- sowie - rückseitenprofils;
- der Vorderkammertiefe, des Vorderkammertiefenprofils,
- der Linsendicke, des Linsendickenprofils, des Linsenvorder- sowie -rückseitenprofils,
- der Glaskörpertiefe, des Glaskörperprofils,
- der Retinaschichtdicke, des Retinaoberflächenprofils,
- der Epitheliumdicke, des Epitheliumprofils, des Epitheliumvorder- und -rückflächenprofils,
- der kornealen Flapdicke, des Flapdickenprofils, des Flapvorder- und — rückseitenprofils, der Flapposition,
- der kornealen Stromadicke, des Stromaprofils, des Stromavorder- und — rückflächenprofils.
- Weitere Messungen können bei postoperativen Nachuntersuchungen nach refraktiver Chirurgie vorgenommen werden.

**Patentansprüche**

1. Verfahren zur Messung optischer Eigenschaften wenigstens zweier voneinander distanzierter Bereiche **(37,39; 79,81; 135/140; 177a-d, 179; 180a-d, 179, 181a-d, 179)** bei einem transparenten und/ oder diffusiven Gegenstand **(5; 75; 109; 121)** mit einer Messzeit im Subsekundenbereich, wobei der Gegenstand **(5; 75; 109; 121; 147)** mit einer der Anzahl Bereiche entsprechenden Anzahl Messstrahlen **(45, 46; 96a, 96b; 71; 101)** bestrahlt wird und jeweils zwei Messstrahlen bis auf eine Bestimmungstoleranz zueinander eine optische Wegdifferenz haben, welche einem geometrischen Abstand in Messstrahlrichtung jeweils zweier Bereiche **(37, 39; 79, 81; 135, 140; 177a-d, 179; 180a-d, 179, 181a-d, 179)** entspricht und jeder von einem der Bereiche **(37, 39; 79, 81; 135, 140; 177a-d, 179; 180a-d, 179, 181a-d, 179)** reflektierte Reflexionsstrahl der Messstrahlen mit einem eine zeitliche, bevorzugt periodische, Weglängenvariation aufweisenden dritten Strahl **(13; 60)** interferierend überlagert und detektiert wird.

2. Verfahren nach Anspruch 1, **dadurch gekennzeichnet, dass** nach Vornahme der Wegdifferenz bzw. -differenzen eine Vereinigung der Messstrahlen zu einer einzigen Strahlkonfiguration mit einer einzigen optischen Achse **(40, 102)** zur Dickenmessung erfolgt und vorzugsweise die Strahlkonfiguration **(71)** über den Gegenstand **(75; 121)** bewegt, insbesonders periodisch über diesen bewegt wird.

3. Verfahren nach Anspruch 1, **dadurch gekennzeichnet, dass** jeweils wenigstens zwei Messstrahlen in einem Abstand nebeneinander verlaufen, um ein Oberflächenprofil zu ermitteln.

4. Verfahren nach einem der Ansprüche 1 bis 3, **dadurch gekennzeichnet, dass** die Messstrahlen im Vergleich zu den Bereichsabständen, insbesondere zu den Bereichsabständen bezüglich einer Referenz **(139)**, eine kurze Kohärenzlänge haben, vor allem die Messstrahlen **(96a, 96b)** jeweils sich voneinander unterscheidende Strahlungsfrequenzen haben, vorzugsweise die Messstrahlen **(45, 46)** jeweils sich voneinander unterscheidende Polarisationszustände haben, insbesondere jeweils ein Messstrahl **(45, 46; 96a, 96b)** auf einen der Bereiche **(37, 39; 79, 81)** fokussiert wird und wahlweise bei der zeitlichen Weglängenvariation des dritten Strahls eine einer vorgegebenen Weglänge entsprechende Referenz definiert wird.

5. Vorrichtung zur Durchführung des Verfahrens nach einem der Ansprüche 1 bis 4 mit einer "Michelson-Interferometer"-artigen optischen Anordnung, in deren Messarm **(3; 55; 157b)** ein optisch transparenter und/oder diffusiver Gegenstand **(5; 75; 109; 121; 147)** mit wenigstens einem reflektierenden Bereich einbringbar ist und deren Referenzarm **(1; 56, 90; 157a)** eine Weglängenvariationseinheit **(17; 65, 66; 161)** aufweist, mit der bevorzugt eine periodische Weglängenänderung für die Strahlung im Referenzarm **(1; 56, 90; 157a)** erzeugbar ist, **gekennzeichnet durch** eine im Messarm **(3; 55; 157b)** vor dem Gegenstand **(5; 75; 109; 121; 147)** angeordnete Umwegeinheit **(29; 70; 95; 120; 163),** mit der wenigstens ein erster Messstrahl mit einer gegenüber wenigstens einem zweiten Messstrahl grösseren Umweg beaufschlagbar ist, wobei ein mit der Umwegeinheit **(29; 70; 95; 120; 163)** erzeugbarer Umweg bis auf eine Bestimmungstoleranz gleich einem Abstand von beim Gegenstand **(5; 75; 109; 121; 147)** auszumessenden Bereichen **(37, 39; 79, 81; 177a-d-179; 180a-d-179, 181a-d-179)**, bevorzugt gleich einen Abstand seitlich benachbarter Bereiche von einer Referenz **(139)** wählbar ist.

6. Vorrichtung nach Anspruch 5, **dadurch gekennzeichnet, dass** die Strahlungsquelle **(7; 50; 149; 191a-e)** der "Michelson-Interferometer"-artigen optischen Anordnung derart ausgebildet ist, dass die Strahlung des von ihr ausgehenden Quellenstrahls **(9; 51)** eine im Vergleich zu den Bereichsabständen kurze Kohärenzlänge hat, die Strahlung wenigstens in zwei unterschiedliche Polarisationsrichtungen aufspaltbar ist und die Umwegeinheit **(29; 70; 163)** wenigstens zwei Strahlteiler **(31, 32; 164, 165)** hat, mit denen der zweite, gegenüber dem ersten Messstrahl eine andere Polarisation aufweisende Messstrahl über den Umweg **(43)** führbar ist.

7. Vorrichtung nach Anspruch 5 oder 6, **dadurch gekennzeichnet, dass** die Strahlungsquelle **(83a, 83b)** der "Michelson-Interferometer"-artigen optischen Anordnung derart ausgebildet ist, dass die Strahlung wenigstens zwei unterschiedliche Strahlungszentrumswellenlängen hat und die Umwegeinheit wenigstens zwei wellenlängen-selektive Strahlteiler **(93, 100)** hat, mit denen der zweite, gegenüber dem ersten Messstrahl eine andere Strahlungsfrequenz aufweisende Messstrahl über den Umweg **(93-94b-97b-99-101)** führbar ist.

8. Vorrichtung nach Anspruch 6 oder 7, **dadurch gekennzeichnet, dass** in der Umwegeinheit **(29; 70; 95; 120)** die geometrisch-optische Länge des Umwegs durch eine Abstandsverstellung eines Umlenkspiegels **(36; 99)** gegenüber einem der Strahlteiler **(32; 100)** veränderbar ist und insbesondere der Strahlteiler **(32; 100)** und jeder diesem zugeordnete Umlenkspiegel **(36; 99)** derart zueinander ausgerichtet sind, dass jeder umgelenkte Messstrahl mit dem nicht umgelenkten eine einzige optische Achse **(40, 102)** im Gegenstand **(5; 75;**

**109; 121)** hat sowie wahlweise je eine Fokussiereinheit **(33, 34; 67, 72, 77, 78; 97a, 97b)** für jeden Messstrahl vorhanden ist, um diesen auf je einen Bereich **(37, 39; 79, 81; 107, 108)** fokussieren zu können.

9. Vorrichtung nach einem der Ansprüche 5 bis 8, **gekennzeichnet durch** eine Speichereinheit **(143),** in der Weglängen der Weglängenvariationseinheit abspeicherbar sind, bei denen eine Interferenz der ersten und dritten sowie zweiten und dritten Messstrahlung erhalten werden, um einen Dickenwert bei vorzugsweise annähernd auf einer Achse fokussierten ersten und zweiten Messstrahlen zu ermitteln bzw. wahlweise ein Oberflächenprofil bei seitlich benachbart liegenden ersten und zweiten Strahlen zu ermitteln.

10. Verwendung des Verfahrens nach Anspruch 1 mit einer Vorrichtung nach Anspruch 5 zur Dicken- und/oder Abstandsmessung von in einem transparenten und/oder diffusiven, bevorzugt örtlich schwer fixierbaren Gegenstand **(5; 75; 109; 121; 147)** vorhandenen optischen Bereichen **(37, 39; 79, 81; 115)** unterschiedlicher optischer Eigenschaften, wie beispielsweise Brechungsindexübergängen, wobei der Umweg in der Umwegeinheit **(29; 70; 95; 118; 120; 163)** bis auf eine Bestimmungstoleranz auf die zu erwartende, zu messende Abmessung eingestellt wird und die mit der Weglängenvariationseinheit **(17; 65, 66; 161)** variierbare Weglänge mindestens der Toleranz entspricht.

11. Verwendung des Verfahrens nach Anspruch 1 mit einer Vorrichtung nach Anspruch 5 zur Profilbestimmung von einem Strahlung reflektierenden, bevorzugt örtlich schwer fixierbaren Gegenstand **(5; 75; 109; 121; 147)**, wobei der Umweg in der Umwegeinheit **(29; 70; 95; 118; 120; 163)** bis auf eine Bestimmungstoleranz auf den zu erwartenden und zu messenden Abstand vorzugsweise von einem Referenzort eingestellt und abgespeichert wird und mit einem vorgängig ermittelten, einen seitlichen Distanzwert aufweisenden Abstandswert zur Profilermittlung verknüpft wird.

12. Verwendung nach Anspruch 11 oder 12, **dadurch gekennzeichnet, dass** der Gegenstand das menschliche Auge **(75; 121; 147)** ist.

**Claims**

1. Method for measuring optical properties of at least two regions (37, 39; 79, 81; 135/140; 177a-d, 179; 180a-d, 179, 181a-d, 179) located at a distance from one another in a transparent and/or diffusive object (5; 75; 109; 121) with a measuring period in the subsecond range, the object (5; 75; 109; 121) being irradiated with a number of measuring beams (45, 46; 96a, 96b; 71; 101) corresponding to the number of regions and each two measuring beams having an optical path difference, within a predetermined tolerance in relation to each other, said optical path difference corresponds to a geometric distance in the measuring beam direction between two respective regions (37, 39; 79, 81; 135, 140; 177a-d, 179; 180a-d, 179; 181a-d, 179) and each reflection beam of the measuring beams reflected from one of the regions (37, 39; 79, 81; 135, 140; 177a-d, 179; 180a-d, 179; 181a-d, 179) being superimposed in an interfering manner with a third beam (13; 60) having a temporal, preferably periodic, optical path length variation, and being.

2. Method according to claim 1, **characterised in that** after carrying out the path difference or differences, for thickness gauging the measuring beams are combined to form a single beam configuration having a single optical axis (40, 102) and preferably the beam configuration (71) is moved over the object (75; 121), in particular is moved periodically thereover.

3. Method according to claim 1, **characterised in that** at least two measuring beams in each case run spaced apart side by side in order to determine a surface profile.

4. Method according to any one of claims 1 to 3, **characterised in that** compared with the spacings of the regions, in particular with the spacings of the regions in respect of a reference (139), the measuring beams have a short coherence length, principally the measuring beams (96a, 96b) each have radiation frequencies differing from one another, preferably the measuring beams (45, 46) each have polarisation states differing from one another, in particular a respective measuring beam (45, 46; 96a, 96b) is focussed on one of the regions (37, 39; 79, 81) and selectively upon temporal path length variation of the third beam a reference corresponding to a predetermined path length is defined.

5. Device for implementing the method according to any one of claims 1 to 4 having a Michelson interferometer-type optical arrangement, into the measuring arm (3; 55; 157b) of which an optically transparent and/or diffusive object (5; 75; 109; 121; 147) having at least one reflecting region can be introduced and the reference arm (1; 56, 90; 157a) of which has a path length variation unit (17; 65, 66; 161), with which preferably a periodic path length modification can be generated for the radiation in the reference arm (1; 56, 90; 157a), **characterised by** a diverting unit (29; 70, 95; 120; 163) arranged in the measuring arm (3; 55; 157b) and in front of the object (5; 75; 109; 121; 147), which unit imposes on at least a first measuring beam a greater diversion compared with at least a second measuring beam, wherein a diversion producible with the diverting unit (29; 70; 95; 120; 163) is selectable, within a predetermined tolerance, to be the same as a spacing of regions (37, 39; 79, 81; 177a-d-179; 180a-d-179, 181a-d-179) to be measured in the object (5; 75; 109; 121; 147), preferably to be the same as a spacing of laterally adjacent regions of a reference (139).

6. Device according to claim 5, **characterised in that** the radiation source (7; 50; 149; 191a-e) of the Michelson interferometer-like optical arrangement is designed so that the radiation of the source beam (9; 51) emitted therefrom has a short coherence length compared with the region spacings, the radiation is splittable at least into two different polarisation directions and the diverting unit (29; 70; 163) has at least two beam splitters (31, 32; 164, 165), with which the second measuring beam having a different polarisation compared with the first measuring beam is guidable via the diversion route.

7. Device according to claim 5 or 6, **characterised in that** the radiation source (83a, 83b) of the Michelson interferometer-like optical arrangement is designed so that the radiation has at least two different radiation centre wavelengths and the diverting unit has at least two wavelength-selective beam splitters (93, 100), with which the second measuring beam having a different radiation frequency compared with the first measuring beam is guidable via the diversion route (93-94b-97b-99-101).

8. Device according to claim 6 or 7, **characterised in that** in the diverting unit (29; 70; 95; 120) the geometrical-optical length of the diversion route is modifiable by a spacing displacement of a tilted mirror (36; 99) relative to one of the beam splitters (32; 100) and in particular the beam splitter (32; 100) and each of the tilted mirrors (36; 99) associated therewith are aligned with respect to one another in such a way that each diverted measuring beam has a single optical axis (40; 102) with the non-diverted measuring beam in the object (5; 75; 109; 121) and selectively a respective focussing unit (33, 34; 67, 72, 77, 78; 97a, 97b) is present for each measuring beam to enable this to be focussed on a respective region (37, 39; 79, 81; 107, 108).

9. Device according to any one of claims 5 to 8, **characterised by** a memory unit (143) in which the path lengths of the path length variation unit are storable, in which an interference of the first and third and second and third measuring radiation are obtained in order, respectively, to determine a thickness value in the case of first and second measuring beams, preferably focussed approximately on one axis, and to determine selectively a surface profile in the case of first and second beams located laterally adjacent.

10. Use of the method according to claim 1 in combination with a device according to claim 5 for measuring the thickness and/or spacing of optical regions (37, 39; 79, 81; 115) having different optical properties, for example refractive index transitions, said optical regions being present in a transparent and/or diffusive and preferably locally not easily fixed object (5; 75; 109; 121; 147), wherein the diversion in the diverting unit (29; 70; 95; 118; 120; 163), within a predetermined tolerance, is set to the anticipated dimension to be measured and the path length variable with the path length variation unit (17; 65, 66; 161) corresponds at least to the tolerance.

11. Use of the method according to claim 1 in combination with a device according to claim 5 for determining the profile of an object (5; 75; 109; 121; 147) reflecting radiation and preferably locally not easily fixed, wherein the diversion of the diverting unit (29; 70; 95; 118; 120; 163), within a predetermined tolerance, is set to the anticipated spacing to be measured, preferably from a reference, and is stored and for determining the profile is combined with a previously determined spacing value having a lateral distance value.

12. Use according to claim 11 or 12, **characterised in that** the object is the human eye (75; 121; 147).

**Revendications**

1. Procédé de mesure des propriétés optiques d'au moins deux secteurs distants l'un de l'autre (37,39 ; 79,81 ; 135/140 ; 177a-d,179 ; 180a-d,179 ; 181a-d,179) dans un objet transparent et/ou diffusant (5 ; 75 ; 109 ; 121) avec un temps de mesure de l'ordre de la fraction de seconde, dans lequel l'objet (5 ; 75 ; 109 ; 121 ; 147) est irradié avec un nombre de faisceaux de mesure (45,46 ; 96a,96b ; 71 ; 101) correspondant au nombre de secteurs et au moins deux faisceaux de mesure ont, à une tolérance de détermination près, une différence de trajet optique qui correspond à un intervalle géométrique dans la direction du faisceau de mesure de deux secteurs (37,39 ; 79,81 ; 135,140 ; 177a-d,179 ; 180a-d, 179 ; 181a-d,179) et chacun des rayons de réflexion des faisceaux de mesure réfléchis par un des secteurs (37,39 ; 79,81 ; 135,140 ; 177a-d, 179 ; 180a-d,179 ; 181a-d,179) est superposé et détecté, à l'aide d'une interférence avec un troisième faisceau (13 ; 60) présentant une variation de longueur de trajet temporaire, de préférence périodique.

2. Procédé selon la revendication 1, **caractérisé en ce que**, après la réalisation de la différence ou des différences de trajet, une unification des faisceaux de mesure est réalisée avec une seule configuration de faisceaux avec un seul axe optique (40,102) pour mesurer l'épaisseur et, de préférence, la configuration des faisceaux (71) est déplacée au-dessus de l'objet (75 ; 121), plus particulièrement déplacée de manière périodique au-dessus de celui-ci.

3. Procédé selon la revendication 1, **caractérisé en ce qu'**au moins deux rayons de mesure sont émis avec un intervalle entre les deux afin de déterminer un profil de surface.

4. Procédé selon l'une des revendications 1 à 3, **caractérisé en ce que** les rayons de mesure ont, par rapport aux intervalles entre les secteurs, plus particulièrement les intervalles entre les secteurs par rapport à une référence (139), une courte longueur de cohérence, avant tout les rayons de mesure (96a,96b) ont des fréquences de rayonnement différentes, de préférence, les rayons de mesure (45,46) ont des états de polarisation différents, plus particulièrement un faisceau de mesure (45,46 ; 96a,96b) est focalisé sur un des secteurs (37,39 ; 79,81) et, de manière facultative, lors de la variation temporaire de longueur de trajet du troisième rayon, une référence correspondant à une longueur de trajet prédéterminée.

5. Dispositif de réalisation du procédé selon l'une des revendications 1 à 4 avec un dispositif optique similaire à un interféromètre de Michelson, dans le bras de mesure (3 ; 55 ; 157b) duquel peut être introduit un objet transparent et/ou diffusif (5 ; 75 ; 109 ; 121 ; 147) avec au moins un secteur réfléchissant et dont le bras de référence (1 ; 56,90 ; 157a) comporte une unité de variation de la longueur du trajet (17 ; 55 ; 66 ; 161) à l'aide de laquelle, de préférence, une variation de la longueur du trajet du rayonnement peut être produite dans le bras de référence (1 ; 56,90 ; 157a), **caractérisé par** une unité de déviation (29 ; 70,95 ; 120 ; 163) disposé dans le bras de mesure (3 ;55 ; 157b) devant l'objet (5 ; 75 ; 109 ; 121 ; 147), à l'aide de laquelle au moins un faisceau de mesure peut être dévié avec un trajet de déviation important par rapport à au moins un deuxième faisceau de mesure, moyennant quoi un trajet de déviation pouvant être réalisé avec l'unité de déviation (29 ; 70 ; 95 ; 120 ; 163) est égal, à une tolérance détermination près, à un intervalle entre les secteurs (37,39 ; 79,81 ; 177a-d-179 ; 180a-d-179,181a-d-179) à mesurer dans un objet (5 ; 75 ;

109 ; 121 ; 147), de préférence égal à un intervalle entre des secteurs voisins latéralement et une référence (139).

6. Dispositif selon la revendication 5, **caractérisé en ce que** la source de rayonnements (7 ; 50 ; 149 ; 191a-e) du dispositif optique similaire à un interféromètre de Michelson est conçue de telle sorte que le rayonnement source (9 ; 51) qui en sort possède une longueur de cohérence courte par rapport aux intervalles entre les secteurs, que le rayonnement puisse être divisé en au moins deux directions de polarisations différentes et que l'unité de déviation (29 ; 70 ; 163) comporte au moins deux séparateurs de faisceaux (31,32 ; 164,165) à l'aide desquels le deuxième faisceau de mesure, ayant une autre polarisation que le premier faisceau de mesure, peut être guidé sur le trajet de déviation (43).

7. Dispositif selon la revendication 5 ou 6, **caractérisé en ce que** la source de rayonnement (83a,83b) du dispositif optique similaire à un interféromètre de Michelson est conçu de telle sorte que le rayonnement comporte au moins deux longueurs d'onde du centre du rayonnement et l'unité de déviation comporte au moins deux séparateurs de faisceaux (93,100) sélectifs en ce qui concerne les longueurs d'onde, à l'aide desquels le deuxième faisceau de mesure, ayant une autre fréquence de rayonnement que le premier faisceau de mesure, peut être guidé sur le trajet de déviation (93-94b-97b-99-101).

8. Dispositif selon la revendication 6 ou 7, **caractérisé en ce que**, dans l'unité de déviation (29 ; 70 ; 95 ; 120), la longueur géométrique et optique du trajet de déviation peut être modifiée par un réglage de l'intervalle d'un miroir de déviation (36 ; 99) par rapport à un des séparateurs de faisceaux (32 ; 100) et, plus particulièrement, le séparateur de faisceaux (32 ; 100) et chacun des miroirs de déviation (36 ; 99) correspondants sont orientés l'un par rapport à l'autre, de telle sorte que le faisceau de mesure dévié ait, avec le faisceau non dévié, un seul axe optique (40,102) dans l'objet (5 ; 75 ; 109 ; 121) ainsi que, de manière facultative, une unité de focalisation (33,34 ; 67,72,77,78 ; 97a,97b) soit prévue pour chaque faisceau de mesure, afin de pouvoir focaliser celui-ci sur chaque secteur (37,39 ; 79,81 ; 107,108).

9. Dispositif selon l'une des revendications 5 à 8, **caractérisé par** une unité de mémoire (143), dans laquelle les longueurs de trajets de l'unité de variation des longueurs de trajets, pour lesquels une interférence du premier et du troisième ainsi que du deuxième et du troisième faisceau de mesure est obtenue, sont mémorisés, afin de déterminer une valeur d'épaisseur, avec un premier et un deuxième faisceau de mesure focalisés approximativement sur un axe ou, de manière facultative, de déterminer un profil de surface lorsque les premier et deuxième faisceaux sont voisins latéralement.

10. Utilisation du procédé selon la revendication 1 avec un dispositif selon la revendication 5 pour la mesure d'épaisseur et/ou d'intervalle de secteurs optiques (37,39 ; 79,81 ; 115), se trouvant dans un objet transparent et/ou diffusif, de préférence dans un objet difficile à fixer localement (5 ; 75 ; 109 ; 121 ; 147), ayant des propriétés optiques différentes comme des transitions d'indice de réfraction, dans laquelle le trajet de déviation dans l'unité de déviation (29 ; 70 ; 95 ; 118 ; 120 ; 63) est ajusté, à la tolérance de détermination près, à la dimension attendue à mesurer et la longueur de trajet pouvant être modifiée à l'aide de l'unité de variation de la longueur de trajet (17 ; 65 ; 66 ; 161) correspond au moins à la tolérance.

11. Utilisation du procédé selon la revendication 1 avec un dispositif selon la revendication 5 pour la détermination du profil d'un objet (5 ; 75 ; 109 ; 121 ; 147) réfléchissant un faisceau et de préférence difficile à fixer localement, dans laquelle le trajet de déviation dans l'unité de déviation (29 ; 70 ; 95 ; 118 ; 120 ; 163) est ajusté à l'intervalle attendu à mesurer, de préférence par rapport à un lieu de référence, à une tolérance de détermination près, mémorisé et relié à une valeur d'intervalle déterminée auparavant et comportant une valeur de distance latérale, pour la détermination du profil.

12. Utilisation selon la revendication 11 ou 12, **caractérisée en ce que** l'objet est l'oeil humain (75 ; 121 ; 147).

Fig. 1

Fig. 2

Fig. 3

Fig. 5

Fig. 4

Fig. 8

Fig. 6

$$\Delta = d_2 - d_1$$

Fig. 11

Fig. 9

Fig. 7

Fig. 10

Fig. 12